# EUROPEAN PATENT SPECIFICATION

(11) **EP 3 067 422 B1**
(45) Date of publication and mention of the grant of the patent: **31.07.2024**
(21) Application number: 16160223.0
(22) Date of filing: 14.03.2016
(51) Int. Cl.: C12N 15/113, C07K 16/24, A61K 39/395, A61P 35/00

(54) **CT-1 INHIBITORS**
CT-1-INHIBITOREN
INHIBITEURS CT-1

(30) Priority: 13.03.2015 TR 201503084
(43) Date of publication of application: 14.09.2016
(73) Proprietor: Sabanci Üniversitesi, 34956 Istanbul (TR)
(72) Inventor: GOZUACIK, Devrim, 34956 Istanbul (TR)
(74) Representative: Mutlu, Aydin

(56) References cited:
- EP-A1- 0 747 480
- WO-A1-2005/030803
- WO-A2-00/43790
- WO-A2-2010/115868
- WO-A2-2011/041582
- WO-A2-2012/154944
- MAR ROYUELA ET AL: "Immunohistochemical analysis of the IL-6 family of cytokines and their receptors in benign, hyperplasic, and malignant human prostate", JOURNAL OF PATHOLOGY, vol. 202, no. 1, 18 January 2004 (2004-01-18), GB, pages 41 - 49, XP055286445, ISSN: 0022-3417, DOI: 10.1002/path.1476
- N. LOPEZ-ANDRES ET AL: "Cardiotrophin 1 Is Involved in Cardiac, Vascular, and Renal Fibrosis and Dysfunction", HYPERTENSION., vol. 60, no. 2, 25 June 2012 (2012-06-25), US, pages 563 - 573, XP055288094, ISSN: 0194-911X, DOI: 10.1161/HYPERTENSIONAHA.112.194407
- HIROTO KINOSHITA ET AL: "Interleukin-6 Mediates Epithelial-Stromal Interactions and Promotes Gastric Tumorigenesis", PLOS ONE, vol. 8, no. 4, 12 April 2013 (2013-04-12), pages e60914, XP055288139, DOI: 10.1371/journal.pone.0060914
- ROY M BREMNES ET AL: "The Role of Tumor Stroma in Cancer Progression and Prognosis Emphasis on Carcinoma-Associated Fibroblasts and Non-small Cell Lung Cancer", J THORAC ONCOL., vol. 6, January 2011 (2011-01-01), pages 209 - 217, XP055288160
- TANIGUCHI KOJI ET AL: "IL-6 and related cytokines as the critical lynchpins between inflammation and cancer", SEMINARS IN IMMUNOLOGY, vol. 26, no. 1, 16 February 2014 (2014-02-16), pages 54 - 74, XP028838309, ISSN: 1044-5323, DOI: 10.1016/J.SMIM.2014.01.001
- MICHAEL FRITZENWANGER ET AL: "Immunomodulatory effects of cardiotrophin-1 on in vitro cytokine production of monocytes & CD4 + T-lymphocytes", INDIAN JOURNAL OF MEDICAL RESEARCH, vol. 136, no. 3, 4 October 2012 (2012-10-04), IN, pages 471 - 476, XP055285852, ISSN: 0971-5916
- CLAUDIA CAPPARELLI ET AL: "Autophagy and senescence in cancer-associated fibroblasts metabolically supports tumor growth and metastasis, via glycolysis and ketone production", CELL CYCLE, vol. 11, no. 12, 15 June 2012 (2012-06-15), US, pages 2285 - 2302, XP055285778, ISSN: 1538-4101, DOI: 10.4161/cc.20718
- HACER EZGI KARAKAS ET AL: "Autophagy and cancer", TURKISH JOURNAL OF BIOLOGY, vol. 38, 24 November 2014 (2014-11-24), TR, pages 720 - 739, XP055285846, ISSN: 1300-0152, DOI: 10.3906/biy-1408-16
- BUSTOS MATILDE ET AL: "Cardiotrophin-1 determines liver engraftment of syngenic colon carcinoma cells through an immune system-mediated mechanism", ONCOIMMUNOLOGY, vol. 1, no. 9, December 2012 (2012-12-01), pages 1527 - 1536, XP055285984, DOI: 10.4161/onci.22504
- VALKENBURG KENNETH C ET AL: "Targeting the tumour stroma to improve cancer therapy.", NATURE REVIEWS. CLINICAL ONCOLOGY 06 2018, vol. 15, no. 6, June 2018 (2018-06-01), pages 366 - 381, XP036507285, ISSN: 1759-4782, DOI: 10.1038/s41571-018-0007-1

## Description

The present invention relates to an inhibitor of CT-1 for use in treatment of tumor growth in stroma environment mediated by fibroblast autophagy.

### Background of the Invention

Cancers are still one of the leading causes of death, despite all the research conducted for decades.

Until relatively recently, many cancer studies focused on examining functional consequences of activating and/or inactivating mutations in critical genes. These studies largely ignored the fact that all cancers contain a diverse population of cells; they comprise genetically altered "tumor" or "cancer" cells as well as other, non-tumorous cell types that are activated and/or recruited to the local microenvironment of tumor cells. Thus the microenvironment of tumor cells is changed in comparison to healthy/ normal tissue.

The local microenvironment of tumor cells which is also referred to as the tumor surrounding tissue or the cancer stroma is composed of non-tumorous cells. The cancer stroma comprises fibroblasts, stem cells, innate and adaptive immune cells, cells that line blood and lymphatic vessels.

Study of the functional link between the tumor cells and the stroma has started in the last decade. Stroma is thought to play an important role in tumor growth, aggressiveness, metastasis and even responsiveness to chemotherapy.

Thus, cancer initiation and progress may be through cell-autonomous behaviour of tumor cells and may also require the support and even contribution of stromal cells.

However there are several unresolved questions about the recruitment of stroma cells by tumor cells. In particular, it is still not clear how one of the most important components of stroma, namely the fibroblasts are recruited, educated or controlled to serve tumor cell interests.

Nutrient catabolism through autophagic degradation is thought to enhance energy source (ketone body and lactate) and possibly cellular building blocks and some secreted factors production by cancer stroma cells, which in turn fuels and supports tumor growth, survival and metastasis. In this stroma supported growth of tumor cells, tumor - derived reactive oxygen species were introduced as major signals that induce autophagy activation and energy source production in stroma cells. Yet, protein factors mediating the tumor - stroma interactions are not known.

The present inventors believe that fibroblast autophagy, i.e. catabolism of fibroblast cells has a vital role in supply of energy and nutrients to tumor cells.

Furthermore, the inventors have some evidence to suggest that tumor cells control their surrounding microenvironment by secreting at least one factor that triggers autophagy.

The present inventors have identified for the first time that tumor cells secrete CT-1 into the extra-cellular environment and this appears to catalyse autophagy.

Autophagy also has a role in promoting the progression of other inflammatory diseases, for example fibrotic diseases in which fibroblasts contribute to the disease progression and pathology.

CT-1 factor with SEQ ID NO:1 was previously reported as being useful in the treatment of heart failure and/or neurological disorders such as peripheral neuropathy, in US 5,571,675. It was also reported as being amplified in tumor cells, in WO00043790.

### Summary of Invention

The present inventors have identified cardiotrophin-1 (CT-1) as a factor that is secreted from cells to mediate autophagy of fibroblasts. Therefore inhibition of extra-cellular CT-1 can ameliorate or halt progression of certain inflammatory diseases.

Thus the present disclosure provides use of a CT-1 inhibitor for treating tumor growth in stroma environment mediated by fibroblast autophagy wherein an increase in CT-1 level of more than 1.4 fold indicates presence of stroma supported tumor growth.

It has been demonstrated that fibroblast autophagy, stimulated by CT-1 factor, is crucial for stroma supported tumor growth. Specifically it has been demonstrated that inhibitors of CT-1, for example an anti-CT-1 antibody, are active in reducing stroma supported tumor growth by inhibiting autophagy in fibroblasts.

Without wishing to be bound by theory, it is believed that when a CT-1 inhibitor for example an anti-CT-1 antibody is used, autophagy in fibroblasts present in the tumor stroma is reduced or prevented. This in turn results in an isolated and nutrient deficient environment for tumor cells, as a result stroma supported tumor growth is reduced.

In one embodiment, tumor is a malignant tumor, i.e. a cancer.

In another aspect, the disclosure provides use of a CT-1 inhibitor for the manufacture of a medicament for use in reducing stroma supported tumor growth.

### Detailed Description

A tumor, as used herein, is an abnormally growing, genetically altered cell. It can be benign, pre-malignant or malignant. In a preferred embodiment, it refers to a malignant tumor, i.e. cancer.

The terms "cancer" and "cancerous" as used herein refers to malignant tumors or describe the physiological condition characterized by unregulated cell growth. Examples of cancer include but are not limited to, carcinoma, lymphoma, blastoma, sarcoma, and leukemia.

In one embodiment, the cancer is carcinoma, i.e. an epithelial cancer.

Carcinoma, as used herein, refers to a type of cancer that develops from epithelial cells. Specifically, a carcinoma begins in a tissue that lines the inner or outer surfaces of the body, and that generally arises from cells originating in the endodermal or ectodermal germ layer during embryogenesis.

Lymphoma, as used herein, refers to a type of cancer that develops from lymphocytes. Blastoma, as used herein, refers to a type of cancer that develops from precursor cells, which are also called blast cells. Blast cell is a partially differentiated, usually unipotent cell that has lost most or all of the stem cell multipotency.

Sarcoma, as used herein, refers to a type of cancer that arises from transformed cells of mesenchymal origin.

Leukemia, as used herein, refers to a type of cancer that begins in the bone marrow and results in high numbers of abnormal white blood cells.

More particular examples of cancers include breast cancer, prostate cancer, colorectal cancer, skin cancer, small-cell lung cancer, non-small cell lung cancer, mesothelioma, gastrointestinal cancer, pancreatic cancer, glioblastoma, vulval cancer, cervical cancer, endometrial carcinoma, ovarian cancer, liver cancer, hepatoma, bladder cancer, kidney cancer, salivary gland carcinoma, thyroid cancer, and various types of head and neck cancer.

In one embodiment, cancer is selected from breast cancer, liver cancer or prostate cancer.

In one embodiment, cancer is breast cancer.

Fibroblasts or fibroblast cells, as used herein, are cells responsible for synthesizing the extracellular matrix and collagen in the structural framework of stroma for animal tissues.

Stroma, as used herein, is the connective, functionally supportive framework of a biological cell, tissue or organ. The stroma comprises fibroblasts, stem cells, innate and adaptive immune cells, cells that line blood and lymphatic vessels.

Tumor stroma, tumor derived stroma cells, or tumor associated stroma cells, as used herein interchangeably, refers to the local microenvironment of tumor cells, composed of non-tumorous cells, in other words it is the tumor surrounding tissue.

Cancer stroma, cancer derived stroma cells or cancer associated stroma cells, as used herein interchangeably, refers to the local microenvironment of cancer cells composed of non-cancerous cells, in other words it is the cancer surrounding tissue.

Autophagy is the basic catabolic mechanism that involves cell degradation of unnecessary or dysfunctional cellular components through the actions of lysosomes. The breakdown of cellular components promotes cellular survival during starvation by maintaining cellular energy levels and anabolic events requiring synthesis.

Fibroblast autophagy, as used herein, refers to autophagy occurring in fibroblast cells.

"Tumor associated" or "tumor derived" fibroblasts, as used herein interchangeably, are fibroblast cells that are present in the tumor stroma. Tumor associated fibroblasts are also called carcinoma associated fibroblasts or CAPs.

In one embodiment, a CT-1 inhibitor is used to reduce or eliminate autophagy in tumor associated fibroblasts, for treating cancer. Surprisingly, this resulted in a decrease in stroma supported tumor growth, whereas not affecting independent tumor growth.

Tumor growth, as used herein, refers to abnormal growth of genetically altered tumor cells.

Stroma supported tumor growth, as used herein, refers to growth of tumor cells in stroma environment, i.e. through enhanced energy and/or building block source (e.g. amino acids, nucleic acids, lipids) source produced in tumor stroma cells. Energy and/or building blocks are produced by autophagic degradation in the stroma cells, particularly fibroblasts.

In contrast, independent tumor growth, as used herein, is the cell-autonomous behaviour of tumor cells without the support or contribution of the tumor stroma.

In one embodiment, tumor growth, as used herein, does not comprise independent tumor growth.

Hence, in one embodiment, the disclosure provides a method for reducing stroma supported tumor growth in a cancer, by inhibiting autophagy in tumor associated fibroblasts, comprising administering a therapeutically effective amount of a CT-1 inhibitor.

CT-1, as used herein, refers to a cytokine, member of IL-6 cytokine family.

The terms "cardiotrophin-1", "CT-1", "CT1", "CT-1 Factor" or "CT-1 polypeptide" as used herein interchangeably, refers to a polypeptide comprising a native sequence polypeptide having the same amino acid sequence as a corresponding CT-1 polypeptide derived from nature, and fragments of such native sequence polypeptides. Such native sequence CT-1 polypeptides can be isolated from nature or, along with the respective fragments, can be produced by recombinant and/or synthetic means.

The term specifically encompasses naturally-occurring truncated or secreted forms (e. g., an extracellular domain sequence), naturally-occurring variant forms (e. g., alternatively spliced forms) and naturally-occurring allelic variants of the CT-1 polypeptide.

Fragments of the respective native polypeptides herein include, but are not limited to, polypeptide variants from which the native N-terminal signal sequence has been fully or partially deleted or replaced by another sequence, and extracellular domains of the respective native sequences, regardless of whether such truncated (secreted) forms occur in nature.

In one embodiment, CT-1, as used herein, is a polypeptide which:
(a) comprises or consists of the amino acid sequence of SEQ ID NO: 1; or
(b) is a derivative having one or more amino acid substitutions, modifications, deletions or insertions relative to the amino acid sequence of SEQ ID NO:1 which retains the activity of the CT-1 polypeptide.

In one embodiment, CT-1 polypeptide is secreted from a cell.

In one embodiment, CT-1 polypeptide is secreted from a tumor cell to tumor stroma.

Hence in one embodiment, a CT-1 inhibitor is used for treating tumors which secrete CT-1 polypeptide extracellularly.

Derivatives of a CT-1 polypeptide, as used herein, refers to biologically active polypeptides having at least 70% identity to the native amino acid sequence shown in Figure 9 (SEQ ID NO:1), more preferably having at least 75%, at least 80%, at least 85%, at least 90%, at least 95% or at least 98% identity. Percentage identity is a well-known concept in the art and can be calculated using, for example but without limitation, the BLAST^{™} software available from NCBI (Altschul, S.F. et al., 1990, J. Mol. Biol. 215:403-410; Gish, W. & States, D.J. 1993, Nature Genet. 3:266-272. Madden, T.L. et al., 1996, Meth. Enzymol. 266:131-141; Altschul, S.F. et al., 1997, Nucleic Acids Res. 25:3389-3402; Zhang, J. & Madden, T.L. 1997, Genome Res. 7:649-656).

The term "polypeptides" includes peptides, polypeptides and proteins. These are used interchangeably unless otherwise specified.

The term 'activity' when used in conjunction with CT-1 refers to the biological activity of CT-1 polypeptide on induction i.e. stimulation, activation or catalysis of autophagy in fibroblasts.

CT-1 is known to activate its receptor expressed at the surface of a human neural cell line by recruiting gp130 and gp130/leukemia inhibitory factor receptor β (Pennica et al., Expression cloning of cardiotrophin 1, a cytokine that induces cardiac myocyte hypertrophy. Proc Natl Acad Sci USA. 1995 Feb. 14;92(4):1142-1146).

CT-1 receptor, or CT-1R as used interchangeably herein, is a receptor containing the gp130 chain, the β subunit of the LIF receptor (LIFRβ) and a third component known as the α subunit of the CT-1 receptor, namely CT-1R α (Robledo et al., Signaling of the cardiotrophin-1 receptor Evidence for a third component, J. Biol. Chem. 1997, 272(8): 4855-4863). The latter component participates in the formation of a three-part complex that confers high sensitivity and specificity to CT-1. Activation of the tripartite CT-1 receptor induces a series of intracellular signals that include the early activation of tyrosine kinases of the JAK family (JAK-1, JAK-2 and Tyk2). The main effectors of the JAKs are the group of cytosolic transcription factors STATs (especially STAT-1 and STAT 3; signal-transducing activators of transcription).

CT-1R, in the context of the present specification is intended to mean each of the subunits separately or together, for example gp130 chain and/or LIFRβ and/or CT-1R α.

So far uncharacterized factors originating from cells expressing and/or secreting CT-1 polypeptide or its stroma may regulate expression of CT-1R on stroma fibroblasts. Alternatively, the process of stroma recruitment during tumor formation may lead to an upregulation of CT-1R in stroma cells. Hence in one embodiment, an increase in expressed and secreted level of CT-1 polypeptide from a cell, may be coupled to increased level of CT-1R in stroma cells.

A CT-1 inhibitor, as used herein, is an agent that inhibits, i.e. reduces, restrains, retards or eliminates the biological activity of CT-1, in particular the activity of CT-1 in fibroblast autophagy.

Inhibitors for use according to the present invention may partially or completely inhibit CT-1 activity. Particularly preferred are agents which interfere with the activity of CT-1 in humans.

In one embodiment, CT-1 inhibitor for use according to the invention inhibits CT-1 activity by modulating or blocking expression or activity of CT1 polypeptide.

Inhibitors according to this embodiment may for example suppress expression of CT1 polypeptide in a cell, inhibit secretion of CT1 from a cell, or block CT1 function by binding CT1 polypeptide.

In a second embodiment, CT1 inhibitor for use according to the invention inhibits CT-1 activity by modulating or blocking expression or activity of CT1 receptor CT1R.

Inhibitors according to this embodiment may for example suppress expression of CT1R, decrease cell surface levels of CT1R or block CT1R activity by binding CT1R.

Modulation, as used herein, is a modulation to inhibit, supress or reduce interaction, expression or activity.

A CT-1 inhibitor, as used herein, is an agent that interacts with, modulates or blocks the expression or activity of a CT-1 polypeptide. Preferably, the CT-1 inhibitor, inhibits, i.e. reduces, restrains, retards or eliminates the biological activity of CT-1, in particular the activity of CT-1 in fibroblast autophagy. Particularly preferred are agents which interfere with the activity of CT-1 in humans.

Modulation, as used herein, is a modulation to inhibit or reduce interaction, expression or activity.

Inhibitors for use according to the present invention may partially or completely inhibit CT-1 activity.

Inhibitors for use in the present invention include without limitation, inhibitors that are capable of interacting with (e.g. binding to, or recognising) CT-1 or the CT-1 receptor (CT-1R) or a nucleic acid molecule encoding CT-1 or CT-1R or are capable of inhibiting or suppressing the expression of CT-1 or CT-1R or are capable of inhibiting the interaction between CT-1 and CT-1R, or are capable of inhibiting secretion of CT-1 from a cell thereby inhibiting the biological activity of CT-1. Such inhibitors may be, without limitation, antibodies, aptamers, nucleic acids (e.g. DNA, RNA, antisense RNA, siRNA, miRNA, shRNA), carbohydrates, lipids, small molecules and other drugs.

In one embodiment, the inhibitor suppresses the expression of CT-1 polypeptide in a cell, for example it down-regulates the CT-1 expression in a cell by modulation of several steps in the gene expression process in a cell, including transcription, RNA splicing, translation and posttranslational modification of a protein.

Examples of suitable inhibitors for inhibiting expression of CT-1 polypeptide in a cell include, but are not limited to, any nucleic acid to alter the expression of CT-1 polypeptide by binding to complementary nucleic acids, i.e. to down-regulate expression of CT-1, specifically, an antisense nucleic acid molecule, a miRNA, a siRNA or shRNA that specifically hybridizes to mRNA encoding CT-1.

In one embodiment, the inhibitor inhibits secretion of CT-1 polypeptide from a cell, for example it binds the immature CT-1 polypeptide preventing its recognition and secretion by the cell membrane.

Immature CT-1 as employed herein refers to a CT-1 polypeptide which is translated but not undergone posttranslational modification yet. An immature CT-1 can differ from a mature protein in that, immature proteins may have additional short N-terminal or C-terminal polypeptides, and/or may lack biochemical functional groups such as acetate, phosphate, lipids or carbohydrates, they may be different in chemical nature of the amino acid, they might have additional disulphide bridges, etc.

Examples of suitable inhibitors for inhibiting secretion of CT-1 polypeptide from a cell include, but are not limited to, antibodies, aptamers, small molecules and other drugs binding an immature CT-1 translated in a cell.

In one embodiment, the inhibitor inhibits activity of CT-1 polypeptide secreted from a cell, for example it binds the mature CT-1 polypeptide.

Mature CT-1 as employed herein refers to CT-1 polypeptide which is translated and undergone posttranslational modification.

Examples of suitable inhibitors for inhibiting activity of CT-1 polypeptide secreted from a cell include, but are not limited to, antibodies, aptamers, synthetic or recombinant functional fragments of the CT-1 receptor that binds to mature form of CT-1 to inhibit CT-1 activity or a small molecule (referred to herein as a chemical entity) or other drug which inhibits the activity of CT-1.

In one embodiment the inhibitor blocks binding of CT-1 to the receptor CT-1R.

In one aspect of this embodiment, the inhibitor is specific to CT-1R, for example binds the active site of the receptor or binds allosterically and thereby prevents the natural ligand i.e. CT-1 binding to the receptor or block generating signalling through the receptor.

In another aspect of this embodiment, the inhibitor is specific to CT-1 and for example binds the site on this ligand that would naturally be involved in receptor binding or alternatively binds allosterically and blocks binding to the receptor or blocks generating a signal through the receptor even if CT-1 is nominally able to bind.

Examples of suitable inhibitors blocking binding of CT-1 to the receptor CT-1R include, but are not limited to, a synthetic or recombinant functional fragment of the CT-1 receptor that binds to CT-1 and interferes with binding to the native CT-1 receptor (for example competitive inhibitors), a synthetic or recombinant functional fragment of CT-1 that binds to CT-1 receptor and interferes with binding to the native CT-1 (for example competitive inhibitors), an antibody or an aptamer that binds to CT-1 or to the CT-1 receptor and interferes with CT-1 receptor-ligand interaction, an antisense nucleic acid molecule, a miRNA, a siRNA or shRNA that specifically hybridizes to mRNA encoding CT-1 or the CT-1 receptor, or a small molecule (referred to herein as a chemical entity) or other drug which inhibits the activity of CT-1 or CT-1 R.

Chemical entity as employed herein refers to a synthetic organic chemical molecule prepared using synthetic chemical methods.

Aptamer, as used herein, refers to oligonucleic acid or peptide molecules that bind to a specific target molecule.

Antibody or immunoglobulin, as used interchangeably herein, includes whole antibodies and any antigen binding fragment or single chains thereof.

The terms "cardiotrophin-1 antibody", "CT-1 antibody", "CT1 antibody", "anti-CT1 antibody" or "CT-1 neutralizing antibody" as used herein interchangeably, refers to specific antibodies for neutralizing, i.e. inhibiting to neutralize the biological effect of CT-1.

The term "antibody" means an immunoglobulin molecule or antigen binding fragment thereof that recognizes and specifically binds to a target, such as a protein, polypeptide, peptide, carbohydrate, polynucleotide, lipid, or combinations of the foregoing through at least one antigen recognition site (also referred to as a binding site) within the variable region of the immunoglobulin molecule. As used herein, the term "antibody" encompasses intact polyclonal antibodies, intact monoclonal antibodies, antibody fragments (such as Fab, Fab', F(ab')2, and Fv fragments), single chain antibody fragments (scFv and disulfide stabilized scFv (dsFv)), multispecific antibodies such as bispecific antibodies generated from at least two different antibodies or multispecific antibodies formed from antibody fragments (see, e.g, PCT Publications WO96/27011, WO2007/024715WO2009018386, WO2009/080251, WO2013006544, WO2013/070565, and WO2013/096291), chimeric antibodies, humanized antibodies, human antibodies, fusion proteins comprising an antigen-binding fragment of an antibody, and any other modified immunoglobulin molecule comprising an antigen-binding fragment so long as the antibodies exhibit the desired biological activity.

Agents that may be suitable inhibitors can be selected from a wide variety of candidate agents. Examples of candidate agents include but are not limited to, nucleic acids including DNA, RNA, specifically miRNA, shRNA, siRNA, lncRNA, carbohydrates, lipids, small molecules and other drugs. Agents can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is suited to peptide and nucleotide libraries, while the other four approaches are applicable to peptide, non-peptide oligomer or small molecule libraries of compounds (Lam, 1997, Anticancer Drug Des. 12:145; US5,738,996; and US5,807,683).

In one example, the inhibitor for use in the present invention may be a nucleic acid. In particular CT-1 or CT-1R nucleic acid molecules may be used as anti-sense molecules, miRNAs, lncRNAs, shRNAs, siRNAs to alter the expression of their respective polypeptides by binding to complementary nucleic acids, i.e. to down-regulate expression of CT-1 or CT-1R. CT-1 or CT-1R nucleic acids may be obtained using standard cloning techniques from, for example genomic DNA or cDNA or can be synthesised using well known and commercially available techniques. The CT-1 or CT-1R nucleic acids may contain one or more nucleotide substitutions, additions or deletions into the nucleotide sequence of a CT-1 or CT-1R nucleic acid. Standard techniques known to those of skill in the art can be used to introduce mutations, including, for example, site-directed mutagenesis and PCR-mediated mutagenesis. An antisense nucleic acid for use according to the present invention includes a CT-1 or CT-1R nucleic acid capable of hybridising by virtue of some sequence complementarity to a portion of an RNA (preferably mRNA) encoding the respective polypeptide. The antisense nucleic acid can be complementary to a coding and/or non-coding region of an mRNA encoding such a polypeptide.

Preferably, the antisense nucleic acids, miRNAs, lncRNAs, shRNAs or siRNAs result in inhibition of the expression of the CT-1 or CT-1R polypeptide. The disclosure also provides the use of nucleic acids comprising nucleotides that are antisense to a gene or cDNA encoding a CT-1 or CT-1R polypeptide for the manufacture of a medicament for reducing or eliminating fibroblast autophagy in the treatment of tumor growth.

Accordingly, there is provided the use of an antibody that inhibits the activity of CT-1 for the manufacture of a medicament for reducing or eliminating fibroblast autophagy in the treatment of tumor growth. Also provided is a method of reducing autophagy for treatment of tumor growth in a subject comprising administering to said subject a therapeutically effective amount of an antibody that inhibits the activity of CT-1.

In one embodiment, the inhibitor for reducing or eliminating fibroblast autophagy in the treatment of tumor growth is an antibody that interacts with (i.e. binds to or recognises) CT-1R and inhibits the activity of CT-1R. In one example the antibodies selectively interact with CT-1R. Selectively interacting with (e.g. recognising or binding to) means that the antibodies have a greater affinity for CT-1R polypeptide than for other polypeptides. Examples of suitable antibodies are those that inhibit the activity of CT-1R by binding to CT-1R in such a manner as to prevent it being biologically active, for example by preventing the binding of CT-1 to its receptor. Another example of suitable antibodies are those that bind to the immature CT-1 in the cell, in such a manner as to prevent it being secreted out of the cell.

In one embodiment the CT-1R antibody is capable of blocking ligand binding to CT-1R. Blocking as employed herein refers to physically blocking such as occluding the receptor but will also include where the antibody or fragments binds an epitope that causes, for example a conformational change which means that the natural ligand to the receptor no longer binds i.e. allosteric blocking. BIAcore is an example of an assay employed to measure binding kinetics, ELISA assays or cell based assays may also be useful. CT-1 is a ligand for CT-1R and the anti-CT-1R antibody blocks binding of CT-1 to CT-1R. The anti-CT-1R antibody also preferably does not cause CT-1R activation, but it may or may not lead to CT-1R internalisation.

Accordingly, there is provided the use of an antibody that inhibits the activity of CT-1R for the manufacture of a medicament for reducing or eliminating fibroblast autophagy in the treatment of tumor growth. Also provided is a method of reducing or eliminating fibroblast autophagy in tumor growth in a subject comprising administering to said subject a therapeutically effective amount of an antibody that inhibits the activity of CT-1R.

Inhibitors for use according to the invention can be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is suited to peptide and nucleotide libraries, while the other four approaches are applicable to peptide, non-peptide oligomer or small molecule libraries of compounds (Lam, 1997, Anticancer Drug Des. 12:145; US5,738,996; and US5,807,683).

In one embodiment, the antibody for use according to the invention is monoclonal, polyclonal, chimeric, humanised or bispecific, or is conjugated to a therapeutic moiety, detectable label, second antibody or a fragment thereof, an effector or reporter molecule, a cytotoxic agent or a cytokine.

CT-1 polypeptides, CT1 receptor polypeptides or cells expressing said polypeptides can be used to produce CT-1 or CT-1R antibodies, e.g. which specifically recognise said CT-1 or CT1 receptor polypeptides. The CT-1 polypeptides may be 'mature' polypeptides or biologically active fragments or derivatives thereof.

CT-1 polypeptides may be prepared by processes well known in the art from genetically engineered host cells comprising expression systems or they may be recovered from natural biological sources.

CT-1 polypeptides may in some instances be part of a larger protein such as a fusion protein for example fused to an affinity tag. A peptide as employed herein refers to an amino acid sequence of 20 amino acids or less. A protein as employed herein refers to a polypeptide with secondary or tertiary structure.

Antibodies generated against these polypeptides may be obtained by administering the polypeptides to an animal, preferably a non-human animal, using well-known and routine protocols, see for example Handbook of Experimental Immunology, D. M. Weir (ed.), Vol 4, Blackwell Scientific Publishers, Oxford, England, 1986. Many warm-blooded animals, such as rabbits, mice, rats, guinea pigs, sheep, goats, cows, horses, monkeys or pigs may be immunised. However, mice, rabbits, pigs and rats are generally more suitable.

CT-1 antibodies include functionally active fragments, derivatives or analogues and may be, but are not limited to, polyclonal, monoclonal, bi-, tri- or tetra-valent antibodies, humanized or chimeric antibodies, single chain antibodies, Fab fragments, Fab' and Fab'₂ fragments, fragments produced by a Fab expression library, anti-idiotypic (anti-Id) antibodies, and epitope-binding fragments of any of the above.

Monoclonal antibody as used herein refers to an antibody obtained from a population of substantially homogeneous antibodies, i.e., the individual antibodies comprising the population are identical except for possible naturally occurring mutations that may be present in minor amounts. They have monovalent affinity, in that they bind to the same epitope.

Poylclonal antibody as used herein refers to a collection of immunoglobulin molecules that react against a specific antigen, each identifying a different epitope.

Monoclonal and polyclonal antibodies may be prepared by any method known in the art. For example the hybridoma technique (Kohler and Milstein, 1975, Nature, 256:495-497), the trioma technique, the human B-cell hybridoma technique (Kozbor et al, 1983, Immunology Today, 4:72) and the EBV-hybridoma technique (Cole et al, Monoclonal Antibodies and Cancer Therapy, p77-96, Alan R Liss, Inc., 1985) can be used in preparation of monoclonal antibodies.

Chimeric antibodies are those antibodies encoded by immunoglobulin genes that have been genetically engineered so that the light and heavy chain genes are composed of immunoglobulin gene segments belonging to different species. These chimeric antibodies are likely to be less antigenic.

Chimeric antibodies may be made by methods known in the art (Milstein et al, 1983, Nature 305:537-539; WO93/08829, Traunecker et al, 1991, EMBO J. 10:3655-3659). Multi-valent antibodies may comprise multiple specificities or may be monospecific (see for example WO92/22853).

In one embodiment the antibody for use in the present invention is humanised. As used herein, the term 'humanised antibody molecule' refers to an antibody molecule wherein the heavy and/or light chain contains one or more CDRs (including, if desired, one or more modified CDRs) from a donor antibody (e.g. a murine monoclonal antibody) grafted into a heavy and/or light chain variable region framework of an acceptor antibody (e.g. a human antibody) (see, e.g. US 5,585,089; WO91/09967). For a review, see Vaughan et al, Nature Biotechnology, 16, 535-539, 1998.

In one embodiment rather than the entire CDR being transferred, only one or more of the specificity determining residues from any one of the CDRs described herein above are transferred to the human antibody framework (see for example, Kashmiri et al., 2005, Methods, 36, 25-34). In one embodiment only the specificity determining residues from one or more of the CDRs described herein above are transferred to the human antibody framework. In another embodiment only the specificity determining residues from each of the CDRs described herein above are transferred to the human antibody framework.

In a humanised antibody for use according to the present invention, the framework regions need not have exactly the same sequence as those of the acceptor antibody. For instance, unusual residues may be changed to more frequently-occurring residues for that acceptor chain class or type. Alternatively, selected residues in the acceptor framework regions may be changed so that they correspond to the residue found at the same position in the donor antibody (see Reichmann et al., 1998, Nature, 332, 323-324). Such changes should be kept to the minimum necessary to recover the affinity of the donor antibody. A protocol for selecting residues in the acceptor framework regions which may need to be changed is set forth in WO91/09967.

When the CDRs or specificity determining residues are grafted, any appropriate acceptor variable region framework sequence may be used having regard to the class/type of the donor antibody from which the CDRs are derived, including mouse, primate and human framework regions.

Suitably, the humanised antibody has a variable domain comprising human acceptor framework regions as well as one or more of CDRs. Thus, provided in one embodiment is humanised antibody which binds human CT-1 wherein the variable domain comprises human acceptor framework regions and non-human donor CDRs.

Examples of human frameworks which can be used in the present invention are KOL, NEWM, REI, EU, TUR, TEI, LAY and POM (Kabat et al., supra). For example, KOL and NEWM can be used for the heavy chain, REI can be used for the light chain and EU, LAY and POM can be used for both the heavy chain and the light chain. Alternatively, human germline sequences may be used; these are available for example at: http://vbase.mrc-cpe.cam.ac.uk/

In a humanised antibody for use according to the present invention, the acceptor heavy and light chains do not necessarily need to be derived from the same antibody and may, if desired, comprise composite chains having framework regions derived from different chains.

Antibodies for use in the invention may also be generated using single lymphocyte antibody methods by cloning and expressing immunoglobulin variable region cDNAs generated from single lymphocytes selected for the production of specific antibodies by for example the methods described by Babcook, J et al, 1996, Proc. Natl. Acad. Sci. USA 93(15):7843-7848, WO92/02551 and WO2004/051268 and WO2004/106377.

Specific as employed herein is intended to refer to an antibody that only recognises the antigen to which it is specific or an antibody that has significantly higher binding affinity to the antigen to which it is specific compared to binding to antigens to which it is non-specific, for example at least 5, 6, 7, 8, 9, 10 times higher binding affinity.

The antibodies for use in the present invention can also be generated using various phage display methods known in the art and include those disclosed by Brinkman et al. (in J. Immunol. Methods, 1995, 182: 41-50), Ames et al (J. Immunol. Methods, 1995, 184:177- 186), Kettleborough et al (Eur. J. Immunol. 1994, 24:952-958), Persic et al. (Gene, 1997 187 9-18), Burton et al (Advances in Immunology, 1994, 57:191-280) and WO 90/02809; WO91/10737; WO92/01047; WO92/18619; WO93/11236; WO95/15982; WO95/20401; and US 5,698,426; 5,223,409; 5,403,484; 5,580,717; 5,427,908; 5,750,753; 5,821,047; 5,571,698; 5,427,908; 5,516,637; 5,780,225; 5,658,727; 5,733,743 and 5,969,108. Techniques for the production of single chain antibodies, such as those described in US 4,946,778 can also be adapted to produce single chain antibodies to CT-1 polypeptides. Also, transgenic mice, or other organisms, including other mammals, may be used to express humanised antibodies.

The antibody used in the present invention may comprise a complete antibody molecule having full length heavy and light chains or a fragment thereof and may be, but are not limited to Fab, modified Fab, Fab', modified Fab', F(ab')2, Fv, single domain antibodies (e.g. VH or VL or VHH), scFv, bi, tri or tetra-valent antibodies, Bis-scFv, diabodies, triabodies, tetrabodies and epitope-binding fragments of any of the above (see for example Holliger and Hudson, 2005, Nature Biotech 23(9):1126-1136; Adair and Lawson, 2005, Drug Design Reviews - Online 2(3), 209-217). The methods for creating and manufacturing these antibody fragments are well known in the art (see for example Verma et al., 1998, Journal of Immunological Methods, 216, 165-181). Other antibody fragments for use in the present invention include the Fab and Fab' fragments described in WO2005/003169, WO2005/003170 and WO2005/003171. Multi-valent antibodies may comprise multiple specificities e.g bispecific or may be monospecific (see for example WO92/22853, WO05/113605, WO2009/040562 and WO2010/035012).

In another embodiment, CT-1 antibodies can be conjugated to a therapeutic agent, such as a cytotoxic agent, a radionuclide or drug moiety to modify a given biological response. The therapeutic agent is not to be construed as limited to classical chemical therapeutic agents. For example, the therapeutic agent may be a drug moiety which may be a protein or polypeptide possessing a desired biological activity. Such moieties may include, for example and without limitation, a toxin such as abrin, ricin A, pseudomonas exotoxin, or diphtheria toxin, a protein such as tumour necrosis factor, α-interferon, β-interferon, nerve growth factor, platelet derived growth factor or tissue plasminogen activator, a thrombotic agent or an anti-angiogenic agent, e.g. angiostatin or endostatin, or, a biological response modifier such as a lymphokine, interleukin-1 (IL-1), interleukin-2 (IL-2), interleukin-6 (IL-6), granulocyte macrophage colony stimulating factor (GM-CSF), granulocyte colony stimulating factor (G-CSF), nerve growth factor (NGF) or other growth factor.

Therapeutic agents also include cytotoxins or cytotoxic agents including any agent that is detrimental to (*e.g.* kills) cells. Examples include taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents also include, but are not limited to, antimetabolites (*e.g*. methotrexate, 6-mercaptopurine, 6-thioguanine, cytarabine, 5-fluorouracil decarbazine), alkylating agents (*e.g.* mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (*e.g.* daunorubicin (formerly daunomycin) and doxorubicin), antibiotics (*e.g.* dactinomycin (formerly actinomycin), bleomycin, mithramycin, anthramycin (AMC), calicheamicins or duocarmycins), and anti-mitotic agents (*e.g.* vincristine and vinblastine).

Other therapeutic moieties may include radionuclides such as ¹¹¹In and ⁹⁰Y, Lu¹⁷⁷, Bismuth²¹³, Californium²⁵², Iridium¹⁹² and Tunsten¹⁸⁸/Rhenium¹⁸⁸; or drugs such as but not limited to, alkylphosphocholines, topoisomerase I inhibitors, taxoids and suramin.

Techniques for conjugating such therapeutic agents to antibodies are well known in the art (see, *e.g.* Amon et al., "Monoclonal Antibodies For Immunotargeting Of Drugs In Cancer Therapy", in Monoclonal Antibodies And Cancer Therapy, Reisfeld et al., eds., 1985 pp. 243-56, ed. Alan R. Liss, Inc; Hellstrom et al., "Antibodies For Drug Delivery", in Controlled Drug Delivery, 2nd Ed., Robinson et al., eds., 1987, pp. 623-53, Marcel Dekker, Inc.; Thorpe, "Antibody Carriers Of Cytotoxic Agents In Cancer Therapy: A Review", in Monoclonal Antibodies '84: Biological And Clinical Applications; Pinchera et al., 1985, eds., pp. 475-506; "Analysis, Results, And Future Prospective Of The Therapeutic Use Of Radiolabelled Antibody In Cancer Therapy", in Monoclonal Antibodies For Cancer Detection And Therapy, Baldwin et al. (eds.), 1985, pp. 303-16, Academic Press; Thorpe et al., 1982 "The Preparation And Cytotoxic Properties Of Antibody-Toxin Conjugates", Immunol. Rev., 62:119-58 and Dubowchik et al., 1999, Pharmacology and Therapeutics, 83, 67-123).

An antibody, optionally conjugated to a therapeutic moiety, can be used therapeutically alone or in combination with chemotherapeutic agents, cytotoxic factor(s) and/or cytokine(s), lymphokines and/or their inhibitors. This combination can for example be provided as an adjuvant therapy. In a preferred embodiment, the CT-1 antibody is used in combination with a cytokine or its inhibitor, selected from but not limited to granulocyte colony-stimulating factor (G-CSF), granulocyte macrophage colony-stimulating factor (GM-CSF), vascular endothelial growth factor (VEGF), interferon alfa, interferon beta, interleukin 2, interleukin 6, interleukin 11, transforming growth factor beta (TGF beta). More preferably the CT-1 antibody is used in combination with, for example as an adjuvant therapy with TGF beta, VEGF and/or IL-6 inhibitors.

The antibodies for use in the invention include analogues and derivatives that are modified, for example but without limitation, by the covalent attachment of any type of molecule. Preferably, said attachment does not impair immunospecific binding. In one aspect, an antibody can be conjugated to a second antibody to form an antibody heteroconjugate (see US 4,676,980).

In one embodiment, derivatives and analogues that are modified are functionally active.

Functionally active derivative or analogue as used herein has the same function of a CT-1 antibody in terms of binding, interacting with or modulating activity of CT-1 polypeptide.

In one embodiment the antibody is provided as CT-1 binding antibody fusion protein which comprises an immunoglobulin moiety, for example a Fab or Fab' fragment, and one or two single domain antibodies (dAb) linked directly or indirectly thereto, for example as described in WO2009/040562, WO2010/035012, WO2011/030107, WO2011/061492 and WO2011/086091.

In one embodiment the fusion protein comprises two domain antibodies, for example as a variable heavy (VH) and variable light (VL) pairing, optionally linked by a disulphide bond.

In one embodiment the Fab or Fab' element of the fusion protein has the same or similar specificity to the single domain antibody or antibodies. In one embodiment the Fab or Fab' has a different specificity to the single domain antibody or antibodies, that is to say the fusion protein are multivalent. In one embodiment a multivalent fusion protein for use according to the present invention has an albumin binding site, for example a VH/VL pair therein provides an albumin binding site.

Where the fusion protein is an antibody fragment linked to an effector or reporter molecule, this may be prepared by standard chemical or recombinant DNA procedures.

Antibody fragments and methods of producing them are well known in the art, see for example Verma et al, 1998, Journal of Immunological Methods, 216, 165-181. Particular examples of antibody fragments for use in the present invention are Fab' fragments which possess a native or a modified hinge region. A number of modified hinge regions have already been described, for example, in US 5,677,425, WO99/15549, and WO98/25971. Further examples of particular antibody fragments for use in the present invention include those described in international patent applications PCT/GB2004/002810, PCT/GB2004/002870 and PCT/GB2004/002871.

Where desired, the antibody fragment may have one or more effector or reporter molecules attached to it. The effector or reporter molecules may be attached to the antibody fragment through any available amino acid side-chain or terminal amino acid functional group located in the fragment, for example any free amino, imino, hydroxyl or carboxyl group.

Preferably antibodies are attached to poly(ethyleneglycol) (PEG) moieties. Preferably, a modified Fab fragment is PEGylated, *i.e.* has PEG (poly(ethyleneglycol)) covalently attached thereto, e.g. according to the method disclosed in EP 0948544 [see also "Poly(ethyleneglycol) Chemistry, Biotechnical and Biomedical Applications", 1992, J. Milton Harris (ed), Plenum Press, New York, "Poly(ethyleneglycol) Chemistry and Biological Applications", 1997, J. Milton Harris and S. Zalipsky (eds), American Chemical Society, Washington DC and "Bioconjugation Protein Coupling Techniques for the Biomedical Sciences", 1998, M. Aslam and A. Dent, Grove Publishers, New York; Chapman, A. 2002, Advanced Drug Delivery Reviews 2002, 54:531-545]. In one embodiment, a PEG modified Fab fragment has a maleimide group covalently linked to a single thiol group in a modified hinge region. A lysine residue may be covalently linked to the maleimide group. To each of the amine groups on the lysine residue may be attached a methoxypoly(ethyleneglycol) polymer having a molecular weight of approximately 20,000 Da. The total molecular weight of the entire effector molecule may therefore be approximately 40,000 Da.

The antibody can be of any class (e.g. IgG, IgE, IgM, IgD and IgA) or subclass of immunoglobulin molecule. In one embodiment the antibody for use in the present invention is of IgG class and may be selected from any of the IgG subclasses IgG1, IgG2, IgG3 or IgG4. Differential ADCC induction by different IgG isotypes is dependent on the affinity of these residues to FcyRs. In humans, IgG1 and IgG3 are known to induce effector functions whereas IgG2 and IgG4 induce effector functions weakly.

The antibody for use in the present invention may include one or more mutations to alter the activity of the antibody. Angal *et al* (Angal S, King DJ, Bodmer MW, Turner A, Lawson AD, Roberts G, Pedley B, and Adair JR 1993. A single amino acid substitution abolishes the heterogeneity of chimeric mouse/human (IgG4) antibody.

It will also be understood by one skilled in the art that antibodies may undergo a variety of posttranslational modifications. The type and extent of these modifications often depends on the host cell line used to express the antibody as well as the culture conditions. Such modifications may include variations in glycosylation, methionine oxidation, diketopiperazine formation, aspartate isomerization and asparagine deamidation. A frequent modification is the loss of a carboxy-terminal basic residue (such as lysine or arginine) due to the action of carboxypeptidases (as described in Harris, RJ. Journal of Chromatography 705:129-134, 1995). In one embodiment the C-terminal lysine of the antibody heavy chain may be absent.

This specific region or epitope of the human CT-1 polypeptide can be identified by any suitable epitope mapping method known in the art. Examples of such methods include screening peptides of varying lengths derived from CT-1 for binding to the antibody employed in the present invention with the smallest fragment that can specifically bind to the antibody containing the sequence of the epitope recognised by the antibody. The CT-1 peptides may be produced synthetically or by proteolytic digestion of the CT-1 polypeptide. Peptides that bind the antibody can be identified by, for example, mass spectrometric analysis. In another example, NMR spectroscopy or X-ray crystallography can be used to identify the epitope bound by an antibody of the present disclosure.

Once identified, the epitopic fragment which binds an antibody of the present disclosure can be used, if required, as an immunogen to obtain additional antibodies which bind the same epitope.

Biological molecules, such as antibodies or fragments, contain acidic and/or basic functional groups, thereby giving the molecule a net positive or negative charge. The amount of overall "observed" charge will depend on the absolute amino acid sequence of the entity, the local environment of the charged groups in the 3D structure and the environmental conditions of the molecule. The isoelectric point (pI) is the pH at which a particular molecule or solvent accessible surface thereof carries no net electrical charge. In one example, the CT-1 antibody and fragments may be engineered to have an appropriate isoelectric point. This may lead to antibodies and/or fragments with more robust properties, in particular suitable solubility and/or stability profiles and/or improved purification characteristics.

It will be appreciated that the affinity of antibodies for use provided by the present invention may be altered using any suitable method known in the art. The present disclosure therefore also relates to variants of the antibody molecules for use according to the present invention, which have an improved affinity for CT-1. Such variants can be obtained by a number of affinity maturation protocols including mutating the CDRs (Yang et al., J. Mol. Biol., 254, 392-403, 1995), chain shuffling (Marks et al., Bio/Technology, 10, 779-783, 1992), use of mutator strains of E. coli (Low et al., J. Mol. Biol., 250, 359-368, 1996), DNA shuffling (Patten et al., Curr. Opin. Biotechnol., 8, 724-733, 1997), phage display (Thompson et al., J. Mol. Biol., 256, 77-88, 1996) and sexual PCR (Crameri et al., Nature, 391, 288-291, 1998). Vaughan et al. (supra) discusses these methods of affinity maturation.

To identify inhibitors of CT-1 activity a number of different approaches may be taken by those skilled in the art. In one example inhibitors are identified by first identifying agents that interact with CT-1, CT-1R or nucleic acids thereof and subsequently testing those agents to identify those that inhibit CT-1 activity.

CT-1 inhibitors that interact with CT-1 or CT-1R may be identified using any suitable method, for example by using a cell-free or cell-based assay system where the CT-1 or CT-1R polypeptide is contacted with a candidate agent and the ability of the candidate agent to interact with the polypeptide is determined. Suitably, the ability of a candidate agent to interact with a CT-1 polypeptide is compared to a reference range or control. If desired, this assay may be used to screen a plurality (e.g. a library) of candidate agents using a plurality of CT-1 or CT-1R polypeptide samples. In one example of a cell free assay, a first and second sample comprising native or recombinant CT-1 or CT-1R polypeptide are contacted with a candidate agent or a control agent and the ability of the candidate agent to interact with the polypeptide is determined by comparing the difference in interaction between the candidate agent and control agent. Suitably, the polypeptide is first immobilized, by, for example, contacting the polypeptide with an immobilised antibody which specifically recognizes and binds it, or by contacting a purified preparation of polypeptide with a surface designed to bind proteins. The polypeptide may be partially or completely purified (e.g. partially or completely free of other polypeptides) or part of a cell lysate. Further, the polypeptide may be a fusion protein comprising the CT-1 or CT-1R polypeptide or a biologically active portion thereof and a domain such as glutathionine-S- transferase or the Fc region of IgGl. Alternatively, the polypeptide can be biotinylated using techniques well known to those of skill in the art (e.g. biotinylation kit, Pierce Chemicals; Rockford, IL). The ability of the candidate agent to interact with the polypeptide can be determined by methods known to those of skill in the art for example, ELISA, BIAcore^{™}, Flow cytometry or fluorescent microvolume assay technology (FMAT). In another example where a cell-based assay is used, a population of cells expressing CT-1 or CT-1R is contacted with a candidate agent and the ability of the candidate agent to interact with the polypeptide is determined. Preferably, the ability of a candidate agent to interact with CT-1 or CT-1R is compared to a reference range or control. The cell, for example, can be of eukaryotic origin (e.g. yeast or mammalian) and can express the CT-1 or CT-1R polypeptide endogenously or be genetically engineered to express the polypeptide. In some instances, the CT-1 or CT-1R polypeptide or the candidate agent is labelled, for example with a radioactive label (such as P, S or I) or a fluorescent label (such as fluorescein isothiocyanate, rhodamine, phycoerythrin, phycocyanin, allophycocyanin, o-phthaldehyde or fluorescamine) to enable detection of an interaction between a polypeptide and a candidate agent. Alternative methods such as ELISA, flow cytometry and FMAT may also be used. Agents which inhibit CT-1 activity may be identified by any suitable method, for example by: (i) comparing the activity of CT-1 in the presence of a candidate agent with the activity of said polypeptide in the absence of the candidate agent or in the presence of a control agent; and (ii) determining whether the candidate agent inhibits activity of CT-1. Such assays can be used to screen candidate agents, in clinical monitoring or in drug development. As described above, agents may be pre-screened where appropriate (e.g. an antibody) to identify agents that interact with CT-1 or CT-1R prior to screening those agents which bind for their ability to inhibit CT-1 activity. In one example a cell-based assay system is used to identify agents capable of inhibiting the activity of CT-1.

In another example inhibitors of CT-1 may down-regulate the expression of the CT-1 polypeptide, for example antisense inhibitors. Such inhibitors may be identified by any method known in the art. In one example such inhibitors are identified in a cell-based assay system. Accordingly, a population of cells expressing a CT-1 polypeptide or nucleic acid are contacted with a candidate agent and the ability of the candidate agent to alter expression of the CT-1 polypeptide or nucleic acid is determined by comparison to a reference range or control. In one example, populations of cells expressing a CT-1 polypeptide are contacted with a candidate agent or a control agent and the ability of the candidate agent to alter the expression of the CT-1 polypeptides or nucleic acids is determined by comparing the difference in the level of expression of the CT-1 polypeptides or nucleic acids between the treated and control populations of cells. If desired, this assay may be used to screen a plurality (e.g. a library) of candidate agents. The cell, for example, can be of eukaryotic origin (e.g. yeast or mammalian) and can express a CT-1 polypeptide endogenously or be genetically engineered to express a CT-1 polypeptide. The ability of the candidate agents to alter the expression of a said polypeptides or nucleic acids can be determined by methods known to those of skill in the art, for example and without limitation, by flow cytometry, radiolabelling, a scintillation assay, immunoprecipitation, Western blot analysis, Northern blot analysis or RT-PCR.

CT-1 inhibitors may be identified or further tested, for example to determine therapeutically effective amounts in one or more animal models. Examples of suitable animals include, but are not limited to, mice, rats, rabbits, monkeys, guinea pigs, dogs and cats. In one example where the agent inhibits the expression of CT-1, a first and second group of mammals are administered with a candidate agent or a control agent and the ability of the candidate agent to inhibit the expression of CT-1 polypeptide or nucleic acid is determined by comparing the difference in the level of expression between the first and second group of mammals. Where desired, the expression levels of the CT-1 polypeptides or nucleic acid in the first and second groups of mammals can be compared to the level of CT-1 polypeptide or nucleic acid in a control group of mammals. The candidate agent or a control agent can be administered by means known in the art (e.g. orally, rectally or parenterally such as intraperitoneally or intravenously, or locally to the site such as intratumorally or intrathecally). Changes in the expression of a polypeptide or nucleic acid can be assessed by the methods outlined above.

In another example, the inhibition of CT-1 activity can be determined by monitoring an amelioration or improvement in disease symptoms, and/or a delayed onset or slowed progression of the disease.

Techniques known to physicians familiar with inflammatory diseases, particularly cancer can be used to determine whether a candidate agent has altered one or more symptoms associated with the disease.

As discussed herein, inhibitors of CT-1 activity find use in the treatment and/or prophylaxis of tumors, more preferably to reduce stroma supported cancer growth by inhibiting fibroblast autophagy. For such use the CT-1 inhibitors will generally be administered in the form of a pharmaceutical composition.

Thus, according to the invention there is provided a pharmaceutical composition comprising a CT-1 inhibitor for use according to the invention and a pharmaceutically acceptable diluent, excipient and /or carrier. The term 'treatment' includes either therapeutic or prophylactic therapy.

Pharmaceutical compositions may also find use as a vaccine and may comprise additional components acceptable for vaccine use and may additionally comprise one or more suitable adjuvants as known to the skilled person.

When a reference is made herein to a method of treating or preventing a disease or condition using a particular inhibitor or combination of inhibitors, it is to be understood that such a reference is intended to include the use of that inhibitor or combination of inhibitors for the manufacture of a medicament for the treatment and/or prophylaxis of tumor growth. CT1 inhibitors for use according to the invention are also referred to as "active agents".

The composition will usually be supplied as part of a sterile, pharmaceutical composition that will normally include a pharmaceutically acceptable carrier. This composition may be in any suitable form (depending upon the desired method of administering it to a patient). The CT-1 inhibitors of use in the invention are preferably administered to a subject orally or intrarectally but may also be administered by a variety of other routes such as transdermally, subcutaneously, intranasally, intravenously, intramuscularly, intratumorally or intrathecally. The most suitable route for administration in any given case will depend on the particular inhibitor, the subject, and the nature and severity of the disease and the physical condition of the subject.

The CT-1 inhibitors of use in the invention may be administered in combination, e.g. simultaneously, sequentially or separately, with one or more other therapeutically active compounds, which may be for example anti-cancer therapies.

In a preferred embodiment, CT-1 inhibitors may be administered in combination with autophagy inhibiting agents selected from the group comprising Bafilomycin A1, Chloroquine, hydroxychloroquine, E-64 protease inhibitor, 3-Methyladenine, Pepstatin A and, small noncoding RNAs.

Pharmaceutical compositions may be conveniently presented in unit dose forms containing a predetermined amount of CT-1 inhibitor of the disclosure per dose. Such a unit may contain for example but without limitation, 1000mg/kg to 0.01mg/kg for example 750mg/kg to 0.1mg/kg, such as 100mg/kg to 1mg/kg depending on the condition being treated, the route of administration and the age, weight and condition of the subject.

Pharmaceutically acceptable carriers for use in the invention may take a wide variety of forms depending, e.g. on the route of administration.

Compositions for oral administration may be liquid or solid. Oral liquid preparations may be in the form of, for example, aqueous or oily suspensions, solutions, emulsions, syrups or elixirs, or may be presented as a dry product for reconstitution with water or other suitable vehicle before use. Oral liquid preparations may contain suspending agents as known in the art. In the case of oral solid preparations such as powders, capsules and tablets, carriers such as starches, sugars, microcrystalline cellulose, granulating agents, lubricants, binders, disintegrating agents, and the like may be included. Due of their ease of administration, tablets and capsules represent the most advantageous oral dosage unit form in which case solid pharmaceutical carriers are generally employed.

In addition to the common dosage forms set out above, CT-1 inhibitors for use according to the invention may also be administered by controlled release means and/or delivery devices. Tablets and capsules may comprise conventional carriers or excipients such as binding agents for example, syrup, acacia, gelatin, sorbitol, tragacanth, or polyvinylpyrrolidone; fillers, for example lactose, sugar, maize-starch, calcium phosphate, sorbitol or glycine; tableting lubricants, for example magnesium stearate, talc, polyethylene glycol or silica; disintegrants, for example potato starch; or acceptable wetting agents such as sodium lauryl sulphate. The tablets may be coated by standard aqueous or non-aqueous techniques according to methods well known in normal pharmaceutical practice. Pharmaceutical compositions of the present disclosure suitable for oral administration may be presented as discrete units such as capsules, cachets or tablets, each containing a predetermined amount of the CT-1 inhibitor as the active agent, as a powder or granules, or as a solution or a suspension in an aqueous liquid, a non-aqueous liquid, an oil-in-water emulsion or a water- in-oil liquid emulsion. Such compositions may be prepared by any of the methods of pharmacy but all methods include the step of bringing into association the CT-1 inhibitor with the carrier, which constitutes one or more necessary ingredients. In general, the compositions are prepared by uniformly and intimately admixing the CT-1 inhibitor with liquid carriers or finely divided solid carriers or both, and then, if necessary, shaping the product into the desired presentation. For example, a tablet may be prepared by compression or moulding, optionally with one or more accessory ingredients.

Pharmaceutical compositions suitable for parenteral administration may be prepared as solutions or suspensions of the CT-1 inhibitors for use according to the invention in water suitably mixed with a surfactant such as hydroxypropylcellulose. Dispersions can also be prepared in glycerol, liquid polyethylene glycols, and mixtures thereof in oils. Under ordinary conditions of storage and use, these preparations contain a preservative to prevent the growth of microorganisms. The pharmaceutical forms suitable for injectable use include aqueous or non-aqueous sterile injection solutions which may contain anti-oxidants, buffers, bacteriostats and solutes which render the composition isotonic with the blood of the intended recipient, and aqueous and non-aqueous sterile suspensions which may include suspending agents and thickening agents. Extemporaneous injection solutions, dispersions and suspensions may be prepared from sterile powders, granules and tablets. Pharmaceutical compositions can be administered with medical devices known in the art. For example, in a preferred embodiment, a pharmaceutical composition for use according to the invention can be administered with a needleless hypodermic injection device, such as the devices disclosed in US 5,399,163; 5,383,851; 5,312,335; 5,064,413; 4,941,880; 4,790,824; or 4,596,556. Examples of well-known implants and modules useful in the present invention include: US 4,487,603, which discloses an implantable micro-infusion pump for dispensing medication at a controlled rate; US 4,486,194, which discloses a therapeutic device for administering medicaments through the skin; US 4,447,233, which discloses a medication infusion pump for delivering medication at a precise infusion rate; US 4,447,224, which discloses a variable flow implantable infusion apparatus for continuous drug delivery; US 4,439,196, which discloses an osmotic drug delivery system having multi-chamber compartments; and US 4,475,196, which discloses an osmotic drug delivery system. Many other such implants, delivery systems, and modules are known to those skilled in the art.

Pharmaceutical compositions adapted for topical administration may be formulated as ointments, creams, suspensions, lotions, powders, solutions, pastes, gels, impregnated dressings, sprays, aerosols or oils, transdermal devices, dusting powders, and the like. These compositions may be prepared via conventional methods containing the CT-1 inhibitor as active agent. Thus, they may also comprise compatible conventional carriers and additives, such as preservatives, solvents to assist drug penetration, emollients in creams or ointments and ethanol or oleyl alcohol for lotions. Such carriers may be present as from about 1 percent up to about 98 percent of the composition. More usually they will form up to about 80 percent of the composition. As an illustration only, a cream or ointment is prepared by mixing sufficient quantities of hydrophilic material and water, containing from about 5-10 percent by weight of the compound, in sufficient quantities to produce a cream or ointment having the desired consistency. Pharmaceutical compositions adapted for transdermal administration may be presented as discrete patches intended to remain in intimate contact with the epidermis of the recipient for a prolonged period of time. For example, the CT-1 inhibitor as the active agent may be delivered from the patch by iontophoresis. For applications to external tissues, for example the mouth and skin, the compositions are preferably applied as a topical ointment or cream. When formulated in an ointment, the CT-1 inhibitor may be employed with either a paraffinic or a water-miscible ointment base. Alternatively, the CT-1 inhibitor may be formulated in a cream with an oil-in-water cream base or a water-in-oil base. Pharmaceutical compositions adapted for topical administration in the mouth include lozenges, pastilles and mouth washes. Pharmaceutical compositions adapted for topical administration to the eye include eye drops wherein the CT-1 inhibitor is dissolved or suspended in a suitable carrier, especially an aqueous solvent. They also include topical ointments or creams as above. Pharmaceutical compositions suitable for rectal administration wherein the carrier is a solid are most preferably presented as unit dose suppositories. Suitable carriers include cocoa butter or other glyceride or materials commonly used in the art, and the suppositories may be conveniently formed by admixture of the combination with the softened or melted capier(s) followed by chilling and shaping moulds. They may also be administered as enemas.

The dosage to be administered of an inhibitor of CT-1 activity will vary according to the particular inhibitor, the type of disease, the subject, and the nature and severity of the disease and the physical condition of the subject, and the selected route of administration; the appropriate dosage can be readily determined by a person skilled in the art. For the treatment and/or prophylaxis of a disease in humans and animals pharmaceutical compositions comprising antibodies can be administered to patients (e.g., human subjects) at therapeutically or prophylactically effective dosages (e.g. dosages which result in inhibition of disease and/or relief of disease symptoms) using any suitable route of administration, such as injection and other routes of administration known in the art for clinical products, such as antibody-based clinical products. The compositions may contain at least 0.05 percent by weight, for example 0.5 to 50 percent by weight such as 1 to 10 percent by weight, or more, by weight, of the inhibitor of the disclosure, depending on the method of administration. It will be recognized by one of skill in the art that the optimal quantity and spacing of individual dosages of an inhibitor of the disclosure will be determined by the nature and extent of the condition being treated, the form, route and site of administration, and the age and condition of the particular subject being treated, and that a physician will ultimately determine appropriate dosages to be used. This dosage may be repeated as often as appropriate. If side effects develop the amount and/or frequency of the dosage can be altered or reduced, in accordance with normal clinical practice. In another example, where the inhibitor is a nucleic acid this may be administered via gene therapy (see for example Hoshida, T. et al, 2002, Pancreas, 25:111-121; Ikuno, Y. 2002, Invest. Ophthalmol. Vis. Sci. 2002 43:2406-2411; Bollard C, 2002, Blood 99:3179- 3187; Lee E, 2001, Mol. Med. 7:773-782). Gene therapy refers to administration to a subject of an expressed or expressible nucleic acid, in one example this is either the CT-1 nucleic acid or portions thereof. Any of the methods for gene therapy available in the art can be used according to the present invention. Delivery of the therapeutic nucleic acid into a patient can be direct in vivo gene therapy (i.e. the patient is directly exposed to the nucleic acid or nucleic acid-containing vector) or indirect *ex vivo* gene therapy (i.e. cells are first transformed with the nucleic acid in vitro and then transplanted into the patient). For example for *in vivo* gene therapy, an expression vector containing the CT-1 nucleic acid may be administered in such a manner that it becomes intracellular, i.e. by infection using a defective or attenuated retroviral or other viral vectors as described, for example, in US 4,980,286 or by Robbins et al, 1998, Pharmacol Ther 80:35-47. The various retroviral vectors that are known in the art are such as those described in Miller et al. (1993, Meth. Enzymol. 217:581-599) which have been modified to delete those retroviral sequences which are not required for packaging of the viral genome and subsequent integration into host cell DNA. Also adenoviral vectors can be used which are advantageous due to their ability to infect non-dividing cells and such high-capacity adenoviral vectors are described in Kochanek (1999, Human Gene Therapy, 10:2451-2459). Chimeric viral vectors that can be used are those described by Reynolds et al. (1999, Molecular Medicine Today, 1:25 -31). Hybrid vectors can also be used and are described by Jacoby et al. (1997, Gene Therapy, 4:1282-1283). Direct injection of naked DNA or through the use of microparticle bombardment (e.g. Gene Gun(R); Biolistic, Dupont) or by coating it with lipids can also be used in gene therapy. Cell-surface receptors/transfecting compounds or through encapsulation in liposomes, microparticles or microcapsules or nanoparticles (e.g. naked or functionalized liposomes, siver sulfate or iron oxide nanoparticles) or by administering the nucleic acid in linkage to a peptide which is known to enter the nucleus or by administering it in linkage to a ligand predisposed to receptor-mediated endocytosis (See Wu and Wu, 1987, J Biol Chem., 262:4429-4432) can be used to target cell types which specifically express the receptors of interest. In *ex vivo* gene therapy, a gene is transferred into cells in vitro using tissue culture and the cells are delivered to the patient by various methods such as injecting subcutaneously, application of the cells into a skin graft and the intravenous injection of recombinant blood cells such as haematopoietic stem or progenitor cells. Cells into which a CT-1 nucleic acid can be introduced for the purposes of gene therapy include, for example, epithelial cells, endothelial cells, keratinocytes, fibroblasts, muscle cells, hepatocytes and blood cells. The blood cells that can be used include, for example, T-lymphocytes, B-lymphocytes, monocytes, macrophages, neutrophils, eosinophils, megakaryotcytes, granulocytes, haematopoietic cells or progenitor cells, and the like.

In one aspect, the pharmaceutical composition for use according to the present invention comprises a CT-1 nucleic acid, said nucleic acid being part of an expression vector that expresses a CT-1 polypeptide or chimeric protein thereof in a suitable host. In particular, such a nucleic acid has a promoter operably linked to the polypeptide coding region, said promoter being inducible or constitutive (and, optionally, tissue-specific). DNA, RNA or anti-RNAs (antagomirs) may also be loaded as native or modified nucleic acid molecules onto above-mentioned gene delivery tools and used for gene therapy.

The invention also provides therapeutic kits, comprising a CT1 inhibitor for use according to the present invention and a carrier in a suitable packaging. CT1 inhibitor for use according to the present invention is selected from any suitable inhibitor described above. In one embodiment the kit comprising CT1 inhibitor for use according to the present invention (for example a CT1 antibody), also comprises one or more therapeutic agent selected from a chemotherapeutic agent, a cytotoxic factor and/or a cytokine, a lymphokine and/or their inhibitors. In a particularly preferred embodiment, a kit comprising a CT1 antibody for use according to the present invention together with TGF beta and/or VEGF and/or IL-6 inhibitors is provided.

In another aspect of the disclosure, detection of a CT-1 level in a subject may be used to identify an appropriate patient population for treatment according to the methods of the invention.

According to the disclosure, when CT-1 is expressed and released from cells, fibroblast autophagy is induced, i.e. autophagy induction capacity is increased. Secretion of CT-1 from a cell may also be coupled to an increased expression of CT-1R.

Thus CT-1 level in a subject may be detected either directly or indirectly, i.e. by detecting CT-1R level or by detecting autophagy induction capacity in a sample.

Diagnosis, as used herein, refers to identification of the disease or the assessment of the severity of the disease, including prognosis and/or monitoring the effectiveness of therapy.

Prognosis, as used herein, refers to the assessment of the outcome of the condition, i.e. to determine the evolution of the condition, and the risk of worsening.

Therapy, as used herein, refers to treatment of a patient to improve health.

Subject, as used herein is a mammal, preferably human. In one embodiment the subject is suspected to have neoplastic cell growth proliferation.

Suitably, the tumor growth is a stroma supported tumor growth.

The detection, as used herein, may be qualitative or quantitative. Hence, detecting may include quantifying where necessary.

The sample derived from the subject, is a biological sample that can be obtained from any source, such as a blood, serum, plasma sample, body fluids, excretions or a tissue sample.

In one embodiment of the disclosure, the sample is blood, serum or plasma sample.

In one embodiment of the disclosure, the sample is a tissue sample, preferably comprising tumor tissue cells.

In one embodiment of the disclosure, the sample is body fluids or excretions preferably lymph, ascites, cerebrospinal fluid, sweat, urine, stool.

In one embodiment the sample is a breast tissue sample.

In another embodiment the sample is a liver tissue sample.

In another embodiment the sample is a colon tissue sample.

In another embodiment the sample is a prostate tissue sample.

In one embodiment of the disclosure, the tissue sample comprises tumor associated stroma cells.

In one embodiment, the captured polypeptide is detected using a directly or indirectly labelled detection reagent which may be immobilised on a solid phase.

A convenient means for detecting/quantifying a CT1 polypeptide involves the use of antibodies. Hence in one embodiment the capture reagent is an antibody.

A CT1 polypeptide can be used as an immunogen to raise antibodies which interact with (bind to or recognise) said polypeptide using methods known in the art. Thus, in a further aspect, the present disclosure provides an antibody that specifically binds to at least one CT1 polypeptide for use in screening for and/or diagnosis of tumor growth in a subject.

CT-1 antibodies can also be used, *inter alia,* for the diagnosis of stroma supported tumor growth by detecting CT-1 expression and secretion from a tumor cell in a biological sample, particularly blood, serum or plasma sample.

Detection of the interaction of an antibody with an antigen can be facilitated by coupling the antibody to a detectable substance for example, but without limitation, an enzyme (such as horseradish peroxidase, alkaline phosphatase, beta-galactosidase, acetylcholinesterase), a prosthetic group (such as streptavidin, avidin, biotin), a fluorescent material (such as umbelliferone, fluorescein, fluorescein isothiocyanate, rhodamine, dichlorotriazinylamine fluorescein, dansyl chloride, phycoerythrin), a luminescent material (such as luminol), a bioluminescent material (such as luciferase, luciferin, aequorin), gold particles, a radioactive nuclide (such as l25I, 131j, Alan, 99Tc), a positron emitting metal or a non- radioactive paramagnetic metal ion (see US 4,741, 900).

Unless the context indicates otherwise, CT 1 nucleic acids include those nucleic acid molecules which may have one or more of the following characteristics and thus may: a) comprise or consist of the DNA sequence of SEQ ID NO : 1 or its RNA equivalent; b) have a sequence which is complementary to the sequences of a) ; c) have a sequence which codes for a CT1 polypeptide; d) have a sequence which shows substantial identity with any of those of a), b) and c) ; or e) is a fragment of a), b), c) or d), which is at least 10 nucleotides in length; and may have one or more of the following characteristics:
1) they may be DNA or RNA or derivatives;
2) they may be single or double stranded;
3) they may be in substantially pure form. Thus, they may be provided in a form which is substantially free from contaminating proteins and/or from other nucleic acids; and
4) they may be with introns or without introns (e. g. as cDNA). Fragments of CT1 nucleic acids are preferably at least 20, at least 30, at least 50, at least 100 or at least 250 nucleotides in length.

The disclosure also provides the use of nucleic acids which are complementary to the CT1 nucleic acids described in (a)- (e) above, and can hybridise to said CT1 nucleic acids. Such nucleic acid molecules are referred to as "hybridising" nucleic acid molecules. For example, but without limitation, hybridising nucleic acid molecules can be useful as probes or primers. Hybridising nucleic acid molecules may have a high degree of sequence identity along its length with a nucleic acid molecule within the scope of (d)- (h) above (e. g. at least 50%, at least 75%, at least 80%, at least 85%, at least 90%, at least 95%, or at least 98% sequence identity). The use of hybridising nucleic acid molecules that can hybridise to any of the nucleic acid molecules discussed above, e. g. in hybridising assays is also covered by the present disclosure.

Unless the context indicates otherwise, CT-1R nucleic acids include those nucleic acid molecules which may have one or more of the following characteristics and thus may: a) comprise or consist of the DNA sequence of SEQ ID NO : 3 and/or SEQ ID NO : 5 or their RNA equivalents; b) have a sequence which is complementary to the sequences of a) ; c) have a sequence which codes for a CT-1R polypeptide; d) have a sequence which shows substantial identity with any of those of a), b) and c) ; or e) is a fragment of a), b), c) or d), which is at least 10 nucleotides in length; and may have one or more of the following characteristics:
1) they may be DNA or RNA;
2) they may be single or double stranded;
3) they may be in substantially pure form. Thus, they may be provided in a form which is substantially free from contaminating proteins and/or from other nucleic acids; and
4) they may be with introns or without introns (e. g. as cDNA).

Fragments of CT-1R nucleic acids are preferably at least 20, at least 30, at least 50, at least 100 or at least 250 nucleotides in length.

The disclosure also provides the use of nucleic acids which are complementary to the CT-1R nucleic acids described in (a)- (e) above, and can hybridise to said CT-1R nucleic acids. Such nucleic acid molecules are referred to as "hybridising" nucleic acid molecules. For example, but without limitation, hybridising nucleic acid molecules can be useful as probes or primers. Hybridising nucleic acid molecules may have a high degree of sequence identity along its length with a nucleic acid molecule within the scope of (d)- (h) above (e. g. at least 50%, at least 75%, at least 80%, at least 85%, at least 90%, at least 95%, or at least 98% sequence identity). The use of hybridising nucleic acid molecules that can hybridise to any of the nucleic acid molecules discussed above, e. g. in hybridising assays is also covered by the present disclosure.

Hybridisation assays can be used for screening or diagnosis of therapy of tumor growth in a subject. Accordingly, such a hybridisation assay comprises:
a) contacting a biological sample, obtained from a subject, containing nucleic acid with a nucleic acid probe capable of hybridising to a CT-1R nucleic acid molecule, under conditions such that hybridisation can occur; and
b) detecting or measuring any resulting hybridisation.

Embodiments are described herein as comprising certain features/elements. The disclosure also extends to separate embodiments consisting or consisting essentially of said features/elements.

Any embodiments specifically and explicitly recited herein may form the basis of a disclaimer either alone or in combination with one or more further embodiments.

The invention will now be described with reference to the following examples, which are merely illustrative and should not in any way be construed as limiting the scope of the present invention.

### Description of Drawings

| | |
|---|---|
| **Figure 1****.A** | is fluorescence micrographs showing GFP-LC3 dot quantification observed in single culture of GFP-LC3 transgenic MEF cells (MEF) and co-cultures of MCF7-RFP cells with MEF (MEF:MCF7) after 48 hours. |
| **Figure 1****.B** | is a bar graph showing GFP-LC3 dot quantification observed in single culture of GFP-LC3 transgenic MEF cells (MEF) and co-cultures of MCF7 cells with MEF (MEF:MCF7) after 24 and 48 hours.. |
| **Figure 2** | is fluorescence micrographs (Fig 2A) and a bar graph (Fig 2B) showing GFP-LC3 dot quantification in MCF7, MDA-MD-231 (MDA) or HUH7 |
| | cancer cell media and in control media CNT (MEF medium) and STV (starvation positive control). |
| **Figure 3** | is a scheme depicting the method used for the unbiased cytokine library screening |
| **Figure 4****.A** | is an image from the immunoblot analysis showing expression of CT-1 in HEK 293T cells and MCF-7, MDA-MB-231 (MDA) cancer cells. |
| **Figure 4****.B** | is microscopy images and a bar graph showing quantification of GFP-LC3 dots representative of autophagy in cells treated with medium from CT-1 overexpressing 293T cells and CT-1 or control antibody. CNT (negative control) and STV (positive control). |
| **Figure 5** | is microscopy images and a bar graph showing recombinant CT-1 induced MEF autophagy in a dose-dependent manner. GFP-LC3 dot counts in MEF are shown after 24h treatment with 5 ng/ml or 25 ng/ml of recombinant CT-1 protein, C (carrier) and NT (not treated). |
| **Figure 6****.A** | is microscopy images of MCF7-RFP cells in single culture (MCF7) or co-culture with wild-type MEF (MEF:MCF7). Control antibody (cnt ab) or CT-1 antibody (CT-1 ab) was added to the culture. |
| **Figure 6****.B** | Growth curves of MCF7-RFP cells in single culture or in co-culture with wild-type MEF. Control antibody (CNT ab) or CT-1 antibody (CT-1 ab) was added to the culture. MCF7-RFP cell numbers per microscope field (under 40x objective) at 24, 48, 72h of culture were shown. |
| **Figure 7** | Cell numbers of MCF7 cells, cultured in the presence of CT-1 antibody (CT-1 ab) or control antibody (CNT ab), at 24, 48 or 72h were shown. |
| **Figure 8** | Microscopy images and growth curves of MCF7-RFP cells in single culture (MCF7) or in co-culture (MEF:MCF7) with wild-type, autophagy competent Atg5(+/+) MEF or autophagy-defective Atg5(-/-) MEF was shown. MCF7-RFP cell numbers per microscope field (under 40x objective) at 24, 48, 72h of culture. |

### SEQUENCES

| | |
|---|---|
| **SEQ ID NO:1** | The amino acid sequence of a native sequence of cardiotrophin-1 (CT-1) isoform 1, Genbank: NP_001321 |
| **SEQ ID NO:2** | The amino acid sequence of a native sequence of cardiotrophin-1 (CT-1) isoform 2, Genbank: NP_001136016 |
| **SEQ ID NO:3** | cDNA sequence of cardiotrophin 1 (CTF1), transcript variant 1 |
| | Genbank: NM_001330.3 cardiotrophin-1 isoform 1 |
| **SEQ ID NO:4** | cDNA sequence of cardiotrophin 1 (CTF1), transcript variant 2 |
| | Genbank: NM_001142544.1 cardiotrophin-1 isoform 2 |
| **SEQ ID NO:5** | The amino acid sequence of a native sequence of CT-1 receptor component Gp130 (also called Interleukin 6 Signal Transducer, IL6ST, IL-6 Receptor Subunit Beta, Oncostatin M Receptor, Interleukin-6 Receptor Subunit Beta, CD130 Antigen) |
| | Uniprot P40189, GenBank: AAA59155.1, NP_002175.2 |
| **SEQ ID NO:6** | cDNA sequence of CT-1 receptor component Gp130 (also called Interleukin 6 Signal Transducer, IL6ST, IL-6 Receptor Subunit Beta, Oncostatin M Receptor, Interleukin-6 Receptor Subunit Beta, CD130 Antigen), GenBank: NM_002184.3 |
| **SEQ ID NO:7** | The amino acid sequence of a native sequence of CT-1 receptor component leukemia inhibitory factor (LIF) receptor precursor, GenBank: NP_001121143.1 |
| **SEQ ID NO:8** | cDNA sequence of CT-1 receptor component leukemia inhibitory factor (LIF) receptor transcript variant 1, GenBank: NM_001127671.1 |
| **SEQ ID NO:9** | cDNA sequence of CT-1 receptor component leukemia inhibitory factor (LIF) receptor transcript variant 2, GenBank: NM_002310.5 |

### EXAMPLES

### 1. Preparation of Cell Cultures and Common Analyses Employed in Examples

Commercially available reagents referred to in the examples were used according to manufacturer's instructions unless otherwise indicated.

### 1.a) Preparation of Cell Lines and Cell Culture

Immortalized mouse embryonic fibroblast cells (MEF) were obtained following transfection with the SV40 whole genome of early passage primary GFP-LC3 transgenic MEF or *Atg5(*+/+*)* MEF or *Atg5(-*/*-)* MEF (As described in previous methods Gozuacik et al., DAP-kinase is a mediator of endoplasmic reticulum stress-induced caspase activation and autophagic cell death, Cell Death Differ 15, 1875-1886, doi:10.1038/cdd.2008.121; Kuma et al., The role of autophagy during the early neonatal starvation period, Nature 432, 1032-1036, doi:10.1038/nature03029 (2004); Hara et al., Suppression of basal autophagy in neural cells causes neurodegenerative disease in mice, Nature 441, 885-889, doi:10.1038/nature04724 (2006)).

MCF7-RFP breast cancer cells were derived from MCF7 cells (ATCC, USA) following lentiviral transduction (see below). MDA-MB-231 (MDA) breast cancer cells stabily expressing mCherry protein (MDA-Cherry, Cat No. AKR 251) were purchased from Cell BioLabs, Inc (USA).

HEK293T, MDA, MCF7 and Huh7 cells were cultured at 37°C under a 5% CO₂ atmosphere in full DMEM culture medium (DMEM medium (Sigma- D5671) supplemented with 10% (v/v) fetal bovine serum (FBS; Biochrom KG, S0115), L-glutamine (Biological Industries, 03-020-1B) and 100U/ml penicillin/streptomycin (Biological Industries, 03-031-1B). GFP-LC3 cells were cultured in full DMEM medium supplemented with 1xMEM non-essential amino asid solution (Gibco, Cat#: 11140-035). *Atg5 (*+/+*)* or *Atg5 (-*/*-)* MEF cells were cultured in MEF medium (full DMEM medium supplemented with 55 µM B-mercaptoethanol). MDA:MEF co-cultures were in full DMEM medium supplemented with 1x MEM non-essential amino acids, and MCF7:MEF co-cultures were in full DMEM medium

### 1.b) Lentiviral Transduction and MCF7 Stable Cell Line Generation

Lentiviruses was produced by the transfection of pRSI9-U6-UbiC-TagRFP-2A-Puro plasmid (Cellecta, USA) together with helper plasmids psPAX2 and pMD2.G (Addgene plasmid # 12260 and #12259) into HEK293T cells. Cell supernatants were harvested at 24h and 48 h after transfection and used to infect cells or stored at -80°C until infection. For stable cell line generation, cells were infected at a 60 % confluence for 24 h with lentiviral supernatants diluted 1:1 with full DMEM culture medium supplemented with 5 µg/mL polybrene (Sigma, H9268). 24 h after the infection, fresh medium was added. MCF7 cells stabily expressing the RFP vector were obtained following 4 weeks puromycin (1 µg/ml) selection.

### 1.c) GFP-LC3 analyses

To detect autophagy activation by GFP-LC3 dot formation test, GFP-LC3 MEF were fixed in cold 4% formaldehyde (Sigma, Catalog #: 15,812-7) for 20 min, washed with PBS, mounted and inspected under 60× magnification using a BX60 fluorescence microscope (Olympus, BX60, Japan). Basal autophagy thresholds were determined as 20 GFP-LC3 dots per control-treated MEF. Dots in at least 150 cells per experimental point were counted and analyzed. Graphs were plotted as percentage of GFP-LC3 dot positive cells over total cell numbers. In some experimets, recombinant cardiotrophin-1 (Sigma, Catalog #: SRP4011) was added to the media at a 5 ng/ml or 25 ng/ml dose.

### 1.d) Co-cultures of cancer cells with MEF.

GFP-LC3 transgenic MEF were seeded on cover slides in 12-well plates to have 1×10⁵ cells/well. After 16 hours, breast cancer cells were plated ontop of fibroblasts to have a 1:10 cancer cell:fibroblast ratio. When indicated, 6 µg/ml CT-1 neutralizing antibody (R&D, Catalog #: MAB2601) or control antibody (Monoclonal anti-Flag M2 antibody, Sigma, F3165) were added to the culture media. At indicated time points, cells were fixed using 4%PFA and nuclei were stained with 1µg/ml Hoechst 33342 dye (Life Technologies; Catalog #: H3570) for 10 min. in PBS and mounted. At least 20 different fields per condition were photographed under fluorescent microscope at a 40x magnification and red cancer cell numbers were determined.

### 1.e) Cell-conditioned medium

Cells (HEK293T, MDA, MCF7 or Huh7 cells) were cultured in a DMEM medium containing 3% FBS with L-glutamine, 100U/ml penicillin and streptomycin at 37°C in a humidified atmosphere containing 5% CO2 (5.5 ml medium/10 cm plate). 72 hours after seeding, culture media were collected and concentrated using Amicon Ultra-4 centrifugal filter units (Milipore-3K cut-off, Catalog #: UFC800396) following centrifugation at 4.000 × g for 40 min. using a swinging bucket rotor. Concentrated media (approx. 300 µl) were diluted in 5 ml full DMEM plus 1x non essential amino acids and added onto GFP-LC3 MEF (1×10⁵ cells/well in 12 well plates and) grown on coverslips.

### 1.f) Screens with a cytokine cDNA library set.

A cytokine cDNA library set that was cloned into mammalian expression vector pCMV-SPORT6 vector was purchased from Thermo Scientific (catalog #: MHS492). Initially, cDNA of 72 different cytokines were first mixed to have pools containing 5-6 different vectors. Pools were transfected to HEK 293T cells and 8 hours post-transfection, fresh full DMEM medium containing 3% FBS was added. 72 hours later, the media were collected and concentrated using Amicon^{®} Ultra- 4 centrifugal filter units as described above. Concentrated media (approx. 300 µl) were diluted in 5 ml fresh media and added onto GFP-LC3 transgenic MEF. 24 hours later, autophagy activation was evaluated under fluorescence microscope. Individual cytokine cDNAs from the pools that were clearly activating GFP-LC3 dot formation were transfected to HEK293T cells, their media were collected, concentrated and tested on GFP-LC3 MEF as described. When indicated, concentrated media were preincubated with 3µg/ml CT-1 neutralizing antibody (R&D, Catalog #: MAB2601) or with 3µg/ml control antibody for 2 hours at 4 degrees before dilution in fresh medium and addition onto cells.

### 1.g) Immunoblot analysis and antibodies.

Cell were lysed in RIPA buffer (50 mM TRIS-HCl pH 7.4, 150 mM NaCl, 1% NP40, 0.25% Na-deoxycholate) supplemented with a complete protease inhibitor cocktail (Roche, 04-693-131-001) and 1mM phenylmethylsulfonyl fluoride (PMDS; Sigma Aldrich, P7626). Cell extracts (30 µg) were separated in 15% SDS-polyacrylamide gels and transferred to PVDF membranes (Roche, 03010040001). Following blockage in 5% nonfat milk in PBST (32 mM Na₂HPO₄, 1.3 mM KCl, 135 mM NaCl and 0.05% Tween 20, pH 7.4) for 1h at RT, membranes were incubated in 3% BSA-PBST solutions containing primary antibodies (ab): anti-ACTB ab (Sigma- Aldrich, A5441, dilution 1:7500) and anti-human CT-1 (R&D, MAB2601, dilution 1:1000). Then, secondary mouse antibodies coupled to horseradish peroxidase (anti-mouse: Jackson Immunoresearch Laboratories, 115035003, dilutions 1:10,000) were applied in 5% milk/PBST for 1 hour at RT and protein bands were revealed using chemiluminescence.

### 1.h) Growth Curve of Breast Cancer Cells.

MCF7 cancer cells were split into 12 well plates as 4×104 cells/well. 6 µg/ml CT-1 neutralization antibody was used. As control, 6 µg/ml monoclonal Flag M2 antibody (Sigma, F3165) was used. After 24 hours, 48 hours and 72 hours, cells were trypsinized and counted with Tryphan Blue (Sigma, T8154) by using the hemocytometer.

### 1.e) Statistical Analyses.

Statistical analyses were performed using Student's two-tailed t-test. Data were presented as means of ± SD of n independent experiments. Values of p< 0.05 were considered as significant.

### 2. Effect of Tumor Stroma on Autophagy Induction

To check whether communication between tumor cells and cells of stroma components such as fibroblasts led to the activation of autophagy, co-cultured GFP-LC3 transgenic MEF cells (GFP-LC3 MEF) were co-cultured with cancer cells and autophagy activation was checked.

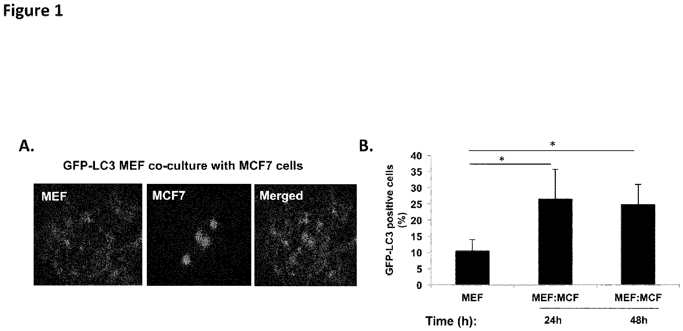

MCF7cancer cells were co-cultured with GFP-LC3 transgenic fibroblasts (GFP-LC3 MEF, green) in a 1:10 ratio for 24h. GFP-LC3 is a commonly used autophagy marker. GFP-LC3 dots were counted in fibroblasts in the beginning and after 24h and 48h co-culture (Fig. 1A and 1B).

Results were expressed as mean ± S.D. of independent experiments, n=3, *p<0.05.

As a result of GFP-LC3 dot quantification, at least a 1.5 fold increase in GFP-LC3 positive cells was observed in co-cultures with cancer cells after 24h and 48h, in Fig. 1.A and Fig.1.B.

These data indicate that under basal conditions, GFP-LC3 shows a diffuse cytosolic and sometimes nuclear appearance. But autophagy induction results in the recruitment of the LC3 protein to autophagic vesicles leading to a punctuate cytoplasmic GFP-LC3 appearance in cells. While mono-cultures of GFP-LC3 MEF showed only basal low levels autophagy activation, co-culture of breast cancer cells with fibroblasts resulted in a significant induction of fibroblast autophagy, especially in fibroblasts that were found in the immediate vicinity of tumor cells (Fig. 1A).

Hence it was concluded that the communication between tumor cells and tumor stroma leads to autophagy activation.

### 3. Analysis of Autophagy induction mediation Mechanism

Next, it was checked whether observed autophagy induction by cancer cells was mediated by soluble secreted factors rather than direct cell-to-cell contacts in co-cultures. To clarify this point, cancer cells of MCF7 and MDA-MB-231 breast cancer cells, HUH7 hepatocellular carcinoma cells and PC3 prostate cancer cells were cultured for 72 hours, their conditioned media was collected and concentrated (appox. 15-20 times concentration) using Amicon centrifugal filter units (3kDa filter cut-off). Concentrated media were added to fresh full DMEM media (see M&M) in order to minimize autophagy induction by nutrient deficiency, and used to culture GFP-LC3 MEF. 24 hours later, autophagy activation was analyzed. MEF starved in EBSS medium for 2 hours (STV) were used as a positive control of autophagy activation, and cells treated with condensed MEF media were used as a negative control (CNT).

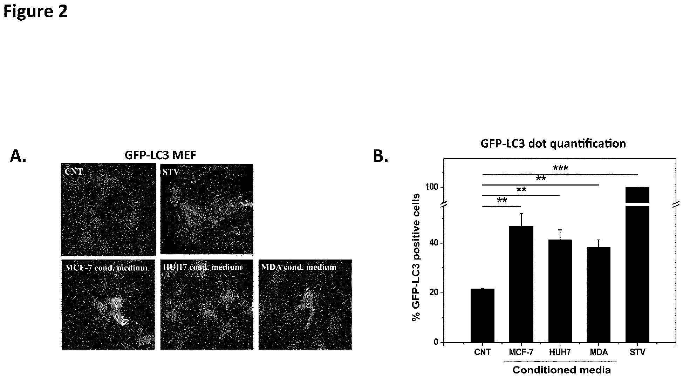

Results of experiments using MCF7, MDA or HUH7-conditioned media were shown in Fig. 2A. Results were expressed as mean ± S.D. of independent experiments, n=3, **<0.01, ***<0.001.

As shown in Fig. 2A, as a result of GFP-LC3 dot quantification, lowest amount of GFP-LC3 positive cells was observed in CNT medium. MCF7, MDA and HUH7 cancer cell media co-cultures showed at least 2 fold increase in GFP-LC3 positive cells, with respect to the CNT. Finally, the highest amount of GFP-LC3 dots was observed in STV medium.

This data indicate that, starvation led to a prominent increase in the number of cells with autophagic vesicles compared to CNT cells. Culture media conditioned by cancer cells significantly activated autophagy in GFP-LC3 MEF, indicating that a soluble factor or factors secreted by cancer cells could strongly induce fibroblast autophagy. The observation was not limited to breast cancer cells, since conditioned media of HUH7 hepatocellular carcinoma cells and PC3 prostate cancer cells (not shown) were also able to activate autophagy in fibroblasts.

Hence it was concluded that, soluble factors secreted from cancer cells activated autophagy in fibroblasts.

### 4. Analysis of Autophagy Inducing Factor

In order to uncover tumor cell-secreted autophagy-inducing factor(s), an unbiased cytokine library screening was performed. A set of cytokine cDNA expression library conssiting of 72 different cytokines was pooled (5-6 cytokines/pool) and transfected to HEK293T cells for expression.

Conditioned cell culture media from HEK292T cells were collected and concentrated using Amicon filters.

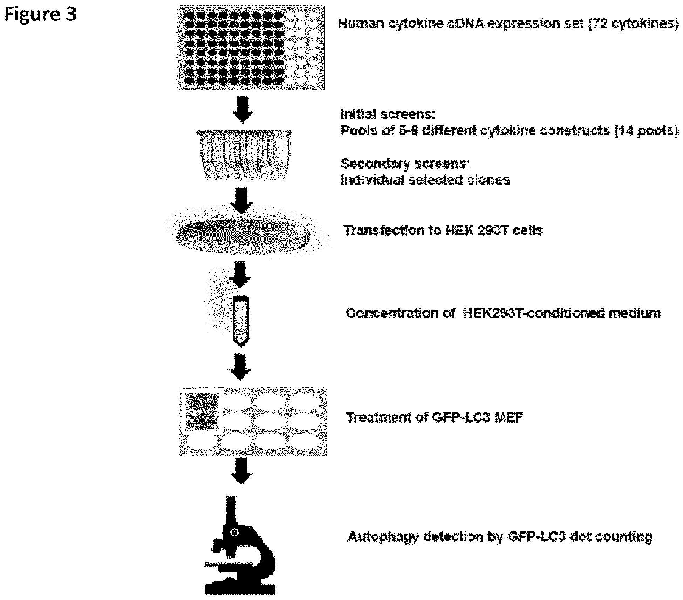

Then, cell-conditioned concentrated media was diluted (approx 20x) by mixing with fresh full DMEM cell culture medium and autophagy activation in GFP-LC3 MEF was checked (Figure 3).

After identifying the pools that potentially contained autophagy stimulating factors, the secondary screens were carried out to identify individual factors that showed an effect on GFP-LC3 MEF autophagy.

In these secondary screens, individual cytokines from pools giving positive results were expressed and similarly tested following the same protocol of conditioning and concentration.

As a result, CT-1 was identified to be a potent cytokine, inducing autophagy in fibroblasts.

### 5. Expression of CT-1 in Tumor Cells

In order to check expression of CT-1 in breast cancer cells, immunoblot analysis was performed.

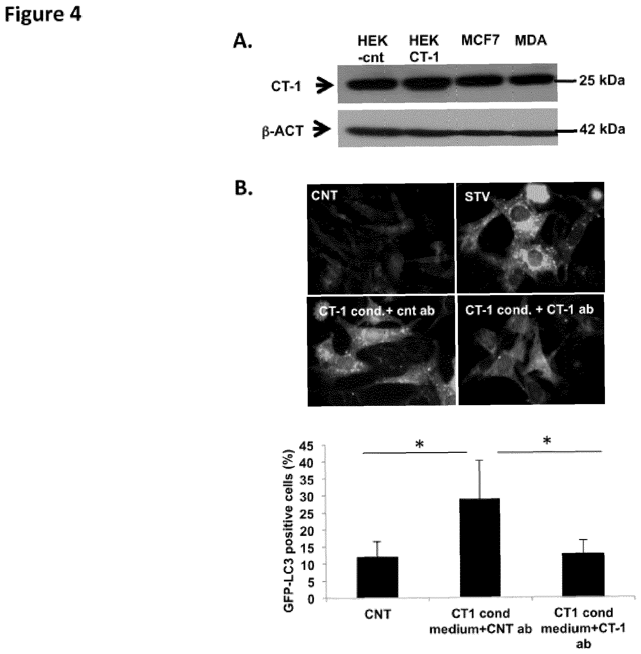

CT-1 cDNA overexpression in HEK293T cells was confirmed using specific antibodies. Transfection of the CT-1 cDNA library vector to HEK293T led to a 1.5-2 folds increase in the expression of the protein. More importantly, CT-1 was clearly expressed in tested cancer cells (Fig. 4A).

### Immunoblot analysis and antibodies

Cell were lysed in RIPA buffer (50 mM TRIS-HCl pH 7.4, 150 mM NaCl, 1% NP40, 0.25% Na-deoxycholate) supplemented with a complete protease inhibitor cocktail (Roche, 04-693-131-001) and 1mM phenylmethylsulfonyl fluoride (PMDS; Sigma Aldrich, P7626). Cell extracts (30 µg) were separated in 15% SDS-polyacrylamide gels and transferred to PVDF membranes (Roche, 03010040001). Following blockage in 5% nonfat milk in PBST (32 mM Na₂HPO₄, 1.3 mM KCl, 135 mM NaCl and 0.05% Tween 20, pH 7.4) for 1h at RT, membranes were incubated in 3% BSA-PBST solutions containing primary antibodies (ab): anti-ACTB ab (Sigma- Aldrich, A5441, dilution 1:7500) and anti-human CT-1 (R&D, MAB2601, dilution 1:1000). Then, secondary mouse antibodies coupled to horseradish peroxidase (anti-mouse: Jackson Immunoresearch Laboratories, 115035003, dilutions 1:10,000) were applied in 5% milk/PBST for 1 hour at RT and protein bands were revealed using chemiluminescence.

### 6. Effect of CT-1 on induction of fibroblast autophagy

To prove that CT-1 was the rate-limiting factor for the observed fibroblast autophagy, GFP-LC3 MEF was treated with conditioned-media from CT-1 overexpressing HEK293T cells following preincubation with a CT-1 neutralizing antibody (CT-1 ab) that binds CT-1 neutralizes its effects or a control antibody (CNT ab):
CT-1 conditioned medium was prepared from HEK3293T cells that were transfected with a mammalian expression vector containing a full lenght CT-1 cDNA sequence. Transfected cells were cultured for 72 hours, their conditioned media containing overexpressed CT-1 was collected and concentrated (appox. 15-20 times concentration) using Amicon centrifugal filter units (3kDa filter cut-off). Concentrated media were added to fresh full DMEM media (see M&M) in order to minimize autophagy induction by nutrient deficiency, and used to culture GFP-LC3 MEF. 24 hours later, autophagy activation was analyzed. Conditioned media from untransfected control HEK293T was used as a negative control medium CNT.

GFP-LC3 MEF cultured in EBSS for 2h was used as a positive control medium STV.

Results were expressed as mean ± SD of independent experiments, n=3, * p<0.05, in Fig.4B.

When the conditioned medium of CT-1 overexpressed HEK 293T cells were added into the medium of GFP-LC3 MEF cells, autophagy is up regulated up to 40%. When neutralizing antibody was incubated with the conditioned medium and then added into the medium of GFP-LC3 MEF cells, the autophagy was down regulated significantly.

This data indicates that, media of recombinant CT-1 protein producing HEK293T cells could induce fibroblast autophagy, and CT-1 neutralizing antibodies significantly block this effect.

As a result, CT-1 was identified as a rate limiting factor for fibroblast autophagy.

### 7. Confirmation of CT-1 protein's effect on autophagy induction

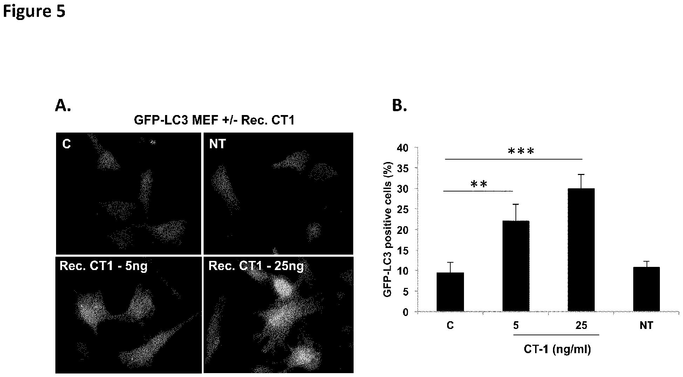

To further confirm that CT-1 protein itself, but not another factor that was present the conditioned medium led to autophagy induction, GFP-LC3 MEF was treated with 5 ng/ml or 25 ng/ml of purified recombinant CT-1 protein and autophagy activation was quantified:
GFP-LC3 MEF were not treated (NT), treated with carrier (Tris buffer, C) or 5 ng/ml or 25 ng/ml of recombinant CT-1 protein (Rec. CT-1). GFP-LC3 dot counts in MEF after 24h treatment with 5 ng/ml or 25 ng/ml of recombinant CT-1 protein. C, carrier. NT, not treated in Fig. 5.

An increase in GFP-LC3 positive cells was observed in conditioned media with recombinant CT-1 protein. Importantly, with respect to the GFP-LC3 MEF medium only treated with carrier (C), a 2 fold increase in autophagy was observed in medium with 5 ng/ml of purified recombinant CT-1, and more than 3.5 fold increase was observed in medium with 25 ng/ml of purified recombinant CT-1.

It was observed that treatment with recombinant CT-1 could potently induce fibroblast autophagy in a dose-dependent manner. At high CT-1 dose (25 ng/ml), the level of induced autophagy was comparable to that obtained using conditioned-media from CT-1 overexpressing HEK293T cells (at least 40%). Therefore, although it is possible that some additional secreted cofactors might be required for the autophagy effect, CT-1 is a major and rate-limiting factor in the communication between cancer cells and fibroblasts.

### 8. Effect of CT-1 mediated autophagy on tumor biology

In order to understand the relevance of CT-1-mediated autophagy signals for tumor biology, tumor cells were cultured alone or in co-culture with fibroblasts, and tumor cell proliferation in the presence of CT-1 neutralizing antibodies or control antibodies were checked.

### 8.a) Role of CT-1 in MEF-supported and -stimulated cancer cell proliferation

Firstly, role of CT-1 in MEF-supported and -stimulated cancer cell proliferation was analysed in single culture (MEF) or co-culture of MEF with MCF7-RFP breast cancer cells prepared in the presence of CT-1 neutralizing antibody (CT-1 ab) or control antibody (CNT ab). All cell nuclei were stained with Hoechst.

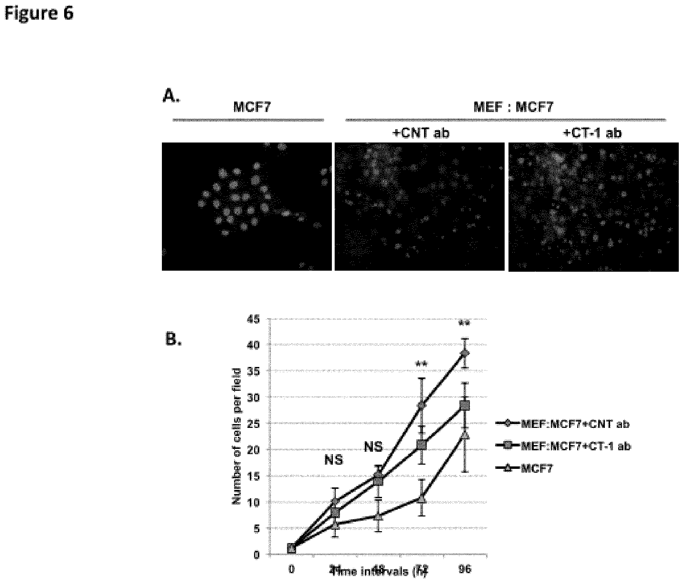

MCF7-RFP cell numbers per microscope area (under 40x objective) at 24, 48, 72h of culture were shown in Fig.6A and 6B.

It was observed that, MCF7 breast cancer cells that were co-cultured with fibroblasts were growing faster compared to cancer cells in single-culture (Fig. 6B, MEF:MCF7 + CNT ab). Strikingly, neutralization of CT-1 under these conditions resulted in a dramatic decrease in the growth rates of MCF7 breast cancer cells (Fig. 6B, MEF:MCF7 + CT-1 ab). On the other hand, single cultures of MCF7 cancer cells without fibroblasts had a lower growth rate with respect to the co-cultures of cancer cells with fibroblasts (Fig. 6B, MCF7).

These results indicated that, fibroblast autophagy was a critical factor in the observed fibroblast-mediated support and stimulation of tumor cell growth. It also indicated that inhibition or neutralization of CT-1 activity led to a decrease in the fibroblast-mediated support and stimulation of tumor cell growth.

### 8.b) Effect of the CT-1 antibody on cancer cell growth

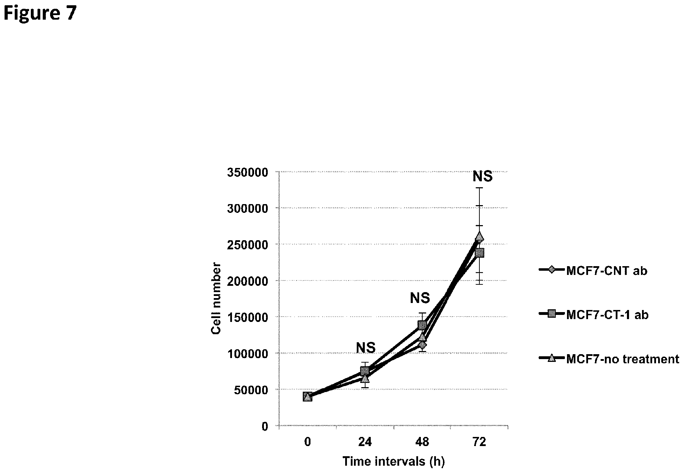

In order to further investigate the effect of CT-1 inhibitors on cancer cell growth, MCF7 cells were cultured in the presence of CT-1 antibody (CT-1 ab) or control antibody (cnt ab). Cell numbers at 24, 48 or 72h culture were shown in Fig. 7a.

Importantly, incubation of tumor cells with CT-1 neutralizing antibodies did not change the growth patterns of either MCF7 (Fig. 7) cells in single culture, excluding autocrine effects of the cytokine (NS: not significant).

Therefore it was concluded that CT-1 had no direct effect on growth of cancer cells in absence of fibroblasts. In co-cultures of fibroblast and tumor cells, the observed effect of fibroblasts on tumor cell growth was dependent on the paracrine activity of CT-1-released from tumor cells.

### 8.c) Role of MEF autophagy in MEF-supported and -stimulated cancer cell proliferation

In the previous examples, it was observed that the CT-1 stimulated autophagy in tumor cells. Previous work from other groups suggested that fibroblast autophagy could sustain tumor cell metabolism through provision metabolites including ketone bodies. To uncover the role of autophagy on fibroblast-supported tumor cell proliferation that was observed under the experimental conditions given in previous examples, tumor cells (MCF7-RFP breast cancer cells) were co-cultured with autophagy competent wild-type fibroblasts (*Atg5(*+/+*)* MEF) or with autophagy-defective *Atg5(-*/*-)* fibroblasts (*Atg5(-*/*-)* MEF).

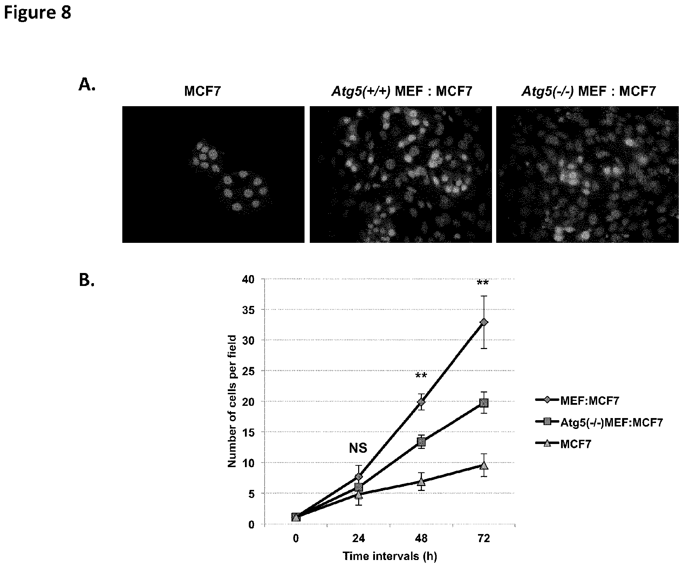

Growth curves of MCF7-RFP cells in culture, analysed by MCF7-RFP cell numbers per microscope area (under 40x objective) at 0, 24, 48, 72h of culture were shown in Figure 8.

As shown in Fig. 8, compared to single cell-type cultures, cell growth was significantly stimulated when MCF7 breast cancer cells were cultured together with *Atg5(*+/+*)* MEF. Yet, *Atg5(-*/*-)* MEF did not have the same effect. Growth rates of MCF7 cells in co-culture with autophagy defective *Atg5(-*/*-)* MEF were closer to that of MCF7 cells cultured alone, and growth advantage provided by the co-culture was minimal (Fig. 8). So it was concluded that, fibroblast autophagy was essential and critical for the stimulation of tumor cell growth in co-cultures.

In conclusion, with the provided examples it was observed that CT-1 factor was secreted from tumor cells to stimulate and activate autophagy in fibroblasts. CT-1 dependent fibroblasts autophagy stimulated and supported tumor cell growth. Blockage of CT-1 and inhibition of CT-1 activity slowed down tumor cell growth. The role of CT-1 in tumor - stroma communication also revealed that proteins or signals mediating this interaction can be exploited for the diagnosis and treatment of tumors and cancers.

## Claims

1. A CT-1 (cardiotrophin-1) inhibitor for use in treatment of tumor growth in stroma environment mediated by fibroblast autophagy wherein an increase in CT-1 level of more than 1.4 fold with respect to standard CT-1 levels in healthy subjects indicates presence of stroma supported tumor growth, wherein the inhibitor is selected from the group comprising a siRNA, shRNA, miRNA, lncRNA suppressing CT1 or CT1R expression, an aptamer, and an antibody blocking CT-1 function selected from a monoclonal, polyclonal, chimeric, humanised or bispecific antibody.

2. The CT-1 inhibitor for use according to claim 1 wherein the stroma supported tumor is a cancer selected from epithelial cancer and breast cancer.

3. The CT-1 inhibitor for use according to claim 1, for treating a tumor which secretes CT1 polypeptide extracellularly.

4. The CT-1 inhibitor for use according to claim 1, for modulating or blocking activity of CT1 polypeptide.

5. The CT-1 inhibitor for use according to claim 1, for modulating or blocking expression of CT1 receptor, CT-1R.

6. The CT-1 inhibitor for use according to claim 1, for modulating or blocking activity of CT-1 receptor, CT-1R.

7. The CT-1 inhibitor for use according to claim 1, wherein the inhibitor is an antibody conjugated to a therapeutic moiety, detectable label, second antibody or a fragment thereof, an effector or reporter molecule, a cytotoxic agent or cytokine.

## Patentansprüche

1. CT-1 (Cardiotrophin-1)-Inhibitor zur Verwendung bei der Behandlung von Tumorwachstum in Stroma-Umgebung, das durch Fibroblasten-Autophagie herbeigeführt wird, wobei ein Anstieg des CT-1-Spiegels um mehr als das 1,4-fache in Bezug auf Standard-CT-1-Spiegel in gesunden Probanden das Vorhandensein von stromagestütztem Tumorwachstum anzeigt, wobei der Inhibitor ausgewählt ist aus der Gruppe umfassend eine siRNA, shRNA, miRNA, die CT1- oder CT1R-Expression unterdrückende lncRNA, ein Aptamer und einen die CT-1-Funktion blockierenden Antikörper ausgewählt aus einem monoklonalen, polyklonalen, chimären, humanisierten oder bispezifischen Antikörper.

2. CT-1-Inhibitor zur Verwendung nach Anspruch 1, der stromagestützte Tumor ein Krebs ist ausgewählt aus Epithelkrebs und Brustkrebs.

3. CT-1-Inhibitor zur Verwendung nach Anspruch 1 zur Behandlung eines Tumors, der CT1-Polypeptid extrazellulär sezerniert.

4. CT-1-Inhibitor zur Verwendung nach Anspruch 1, zur Modulation oder Blockierung der Aktivität des CT1-Polypeptids.

5. CT-1-Inhibitor zur Verwendung nach Anspruch 1, zur Modulation oder Blockierung der Expression des CT1-Rezeptors, CT-1R.

6. CT-1-Inhibitor zur Verwendung nach Anspruch 1, zur Modulation oder Blockierung der Aktivität des CT-1-Rezeptors, CT-1R.

7. CT-1-Inhibitor zur Verwendung nach Anspruch 1, wobei der Inhibitor ein Antikörper ist, der mit einer therapeutischen Einheit, einem nachweisbaren Marker, einem zweiten Antikörper oder einem Fragment davon, einem Effektor- oder Reportermolekül, einem zytotoxischen Mittel oder einem Zytokin konjugiert ist.

## Revendications

1. Inhibiteur de CT-1 (cardiotrophine 1) pour utilisation dans le traitement de croissance tumorale dans un environnement stromal médié par autophagie des fibroblastes, une augmentation du taux de CT-1 de plus de 1,4 fois par rapport aux taux normaux de CT-1 chez des sujets sains indiquant la présence de croissance tumorale soutenue par le stroma, l'inhibiteur étant choisi dans l'ensemble comprenant un ARNsi, ARNsh, ARNmi, ARNInc supprimant l'expression de CT1 ou CT1R, un aptamère, et un anticorps bloquant la fonction de CT-1, choisi parmi un anticorps monoclonal, polyclonal, chimérique, humanisé ou bispécifique.

2. Inhibiteur de CT-1 pour utilisation selon la revendication 1, dans lequel la tumeur soutenue par le stroma est un cancer choisie parmi un cancer épithélial et un cancer du sein.

3. Inhibiteur de CT-1 pour utilisation selon la revendication 1, pour le traitement d'une tumeur qui sécrète extracellulairement le polypeptide CT1.

4. Inhibiteur de CT-1 pour utilisation selon la revendication 1, pour moduler ou bloquer l'activité du polypeptide CT1.

5. Inhibiteur de CT-1 pour utilisation selon la revendication 1, pour moduler ou bloquer l'expression du récepteur de CT1, CT-1R.

6. Inhibiteur de CT-1 pour utilisation selon la revendication 1, pour moduler ou bloquer l'activité du récepteur de CT1, CT-1R.

7. Inhibiteur de CT-1 pour utilisation selon la revendication 1, dans lequel l'inhibiteur est un anticorps conjugué à un fragment thérapeutique, un marqueur détectable, un second anticorps ou un fragment de celui-ci, une molécule effectrice ou rapporteuse, un agent cytotoxique ou une cytokine.
